(19) Europäisches Patentamt
European Patent Office
Office européen des brevets

(11) Veröffentlichungsnummer: **0 001 769**
**A1**

(12) # EUROPÄISCHE PATENTANMELDUNG

(21) Anmeldenummer: **78101135.8**

(22) Anmeldetag: **13.10.78**

(51) Int. Cl.[2]: **C 07 D 211/90**
A 61 K 31/44, C 07 D 401/04
C 07 D 401/12
//C07D401/00, C07D405/00,
C07D409/00, C07D413/00,
C07D417/00

(30) Priorität: **22.10.77 DE 2747513**

(43) Veröffentlichungstag der Anmeldung:
**16.05.79 Patentblatt 79/10**

(84) Benannte Vertragsstaaten:
**BE CH DE FR GB NL**

(71) Anmelder: **Bayer Aktiengesellschaft**
**Zentralbereich Patente,Marken und Lizenzen Bayerwerk**
**D-5090 Leverkusen 1(DE)**

(72) Erfinder: **Wehinger, Egbert, Dr.**
**Donnenbergerstrasse 90**
**D-5620 Velbert 15(DE)**

(72) Erfinder: **Bossert, Friedrich, Dr.**
**Claudiusweg 7**
**D-5600 Wuppertal 1(DE)**

(72) Erfinder: **Franckowiak, Gerhard, Dr.**
**Pahlkestrasse 17**
**D-5600 Wuppertal 1(DE)**

(72) Erfinder: **Heise, Arend, Dr.**
**Moebeck 50**
**D-5600 Wuppertal 1(DE)**

(72) Erfinder: **Kazda, Stanislav, Dr.**
**Pahlkestrasse 55N**
**D-5600 Wuppertal 1(DE)**

(72) Erfinder: **Meyer, Horst, Dr.**
**Theodor-Heuss-Strasse 110**
**D-5600 Wuppertal 1(DE)**

(72) Erfinder: **Stoepel, Kurt, Dr.**
**In den Birken 69**
**D-5600 Wuppertal 1(DE)**

(72) Erfinder: **Towart, Robertson, Dr.**
**Claudiusweg 9**
**D-5600 Wuppertal 1(DE)**

(54) **1,4-Dihydropyridincarbonsäureester mit schwefelhaltigen Estergruppen, ihre Herstellung und ihre Verwendung als Arzneimittel.**

(57) 1,4-Dihydropyridin - 3-carbonsäureester mit-schwefelhaltigen Estergruppen werden nach verschieden Verfahren hergestellt. Die Verbindungen besitzen pharmakologische Eigenschaften, insbesondere eine kreislaufbeeinflussende Wirkung und können als antihypertensive Mittel Verwendung finden.

EP 0 001 769 A1

0001769



BEZEICHNUNG GEÄNDERT
siehe Titelseite

BAYER AKTIENGESELLSCHAFT
Zentralbereich
Patente, Marken und Lizenzen

5090 Leverkusen, Bayerwerk
KS-by
Ib (Pha)

Dihydropyridine mit schwefelhaltigen Estergruppierungen

Die vorliegende Erfindung betrifft neue 1.4-Dihydropyridine mit schwefelhaltigen Estergruppierungen, mehrere Verfahren zu ihrer Herstellung sowie ihre Verwendung als Arzneimittel, insbesondere als kreislaufbeeinflussende Wirkstoffe.

Es ist bereits bekannt geworden,daß man 1,4-Dihydro-2,6-dimethyl-4-phenyl-pyridin-3,5-dicarbonsäurediäthylester erhält, wenn man Benzylidenacetessigsäureäthylester mit β-Amino-crotonsäureäthylester oder Acetessigsäureäthylester und Ammoniak umsetzt (Knoevenagel, Ber. dtsch. chem. Ges. 31, 743 (1898) ).

Weiterhin ist bekannt, daß bestimmte 1,4-Dihydropyridine interessante pharmakologische Eigenschaften aufweisen (F.Bossert, W. Vater, Naturwissenschaften 58, 578 (1971) ).

Die vorliegende Erfindung betrifft neue 1,4-Dihydropyridine mit schwefelhaltigen Estergruppierungen der Formel (I),

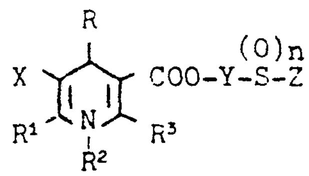

(I)

in welcher

R für einen Arylrest oder für einen Thienyl-, Furyl-, Pyrryl-, Pyrazolyl-, Imidazolyl-, Oxazolyl-, Isoxazolyl-, Thiazolyl-, Pyridyl-, Pyridazinyl-, Pyrimidyl-, Pyrazinyl-, Chinolyl-, Isochinolyl-, Indolyl- Benzimidazolyl-, Chinazolyl- oder Chinoxalylrest steht, wobei der Arylrest sowie die Heterocyclen gegebenenfalls 1 bis 3 gleiche oder verschiedene Substituenten aus der Gruppe Phenyl, Alkyl, Alkenyl, Alkinyl, Alkoxy, Alkenoxy, Alkinoxy, Alkylen, Dioxyalkylen, Halogen, Trifluormethyl, Trifluormethoxy, Hydroxy, Amino, Alkylamino, Nitro, Cyano, Azido, Carboxy, Carbalkoxy, Carbonamido, Sulfonamido oder $SO_m$-Alkyl (m = 0 bis 2) enthalten,

$R^1$ und $R^3$ gleich oder verschieden sind und für Wasserstoff, einen geradkettigen oder verzweigten Alkylrest, einen Arylrest oder einen Aralkylrest stehen,

$R^2$ Wasserstoff oder einen geradkettigen oder verzweigten

BAD ORIGINAL

Alkylrest bedeutet, der gegebenenfalls durch ein oder zwei Sauerstoffatome in der Alkylkette unterbrochen ist, oder für einen Aryl- oder Aralkylrest steht,

Y eine gegebenenfalls durch Alkyl substituierte Alkylengruppe darstellt,

n 0, 1 oder 2 bedeutet,

Z für einen geradkettigen, verzweigten oder cyclischen, gesättigten oder ungesättigten aliphatischen Kohlenwasserstoffrest steht, der gegebenenfalls durch ein oder zwei Sauerstoffatome in der Kette unterbrochen ist und/oder der gegebenenfalls substituiert ist durch eine Phenoxy- oder Phenylgruppe, welche ihrerseits gegebenenfalls substituiert sind durch Halogen, Cyano, Dialkylamino, Alkyl, Alkoxy, Trifluormethyl oder Nitro, oder der gegebenenfalls substituiert ist durch eine α-, ß- oder γ-Pyridylgruppe, und

X für die Gruppe $-COR^4$, worin $R^4$ gegebenenfalls substituiertes Alkyl, Aryl oder Aralkyl oder eine Amino-, Monoalkylamino- oder Dialkylaminogruppe bedeutet,
oder
für die Gruppe $-COOR^5$, wobei $R^5$ einen geradkettigen, verzweigten oder cyclischen, gesättigten oder ungesättigten Kohlenwasserstoffrest darstellt, der gegebenenfalls durch 1 oder 2 Sauerstoffatome in der Kette unterbrochen ist, und/oder der gegebenenfalls substituiert ist durch eine Phenyl-, Phenoxy-, Phenylthio- oder Phenylsulfonylgruppe, welche ihrerseits wieder durch Halogen, Cyano, Dialkylamino, Alkoxy, Alkyl, Trifluormethyl oder Nitro substituiert sein können,

oder der gegebenenfalls durch eine α-, β- oder γ-Pyridylgruppe oder durch eine Aminogruppe substituiert ist, wobei diese Aminogruppe zwei gleiche oder verschiedene Substituenten aus der Gruppe Alkyl, Alkoxyalkyl, Aryl und Aralkyl trägt und wobei diese Substituenten gegebenenfalls mit dem Stickstoffatom einen 5- bis 7-gliedrigen Ring bilden, der als weiteres Heteroatom ein Sauerstoff- oder Schwefelatom oder die N-Alkyl-Gruppierung enthalten kann,

oder

für die Gruppe $-COO-Y'-S(O)_{n}'-Z'$, wobei diese Gruppe mit $-COO-Y-S(O)_{n}-Z$ gleich oder verschieden sein kann und wobei die Definitionen von Y', n' und Z' denen von Y, n und Z entsprechen,

oder

für die Gruppe $-S(O)_{r}-R^{6}$ steht, in der r = 0, 1 oder 2 bedeutet und $R^{6}$ einen geradkettigen, verzweigten oder cyclischen, gesättigten oder ungesättigten aliphatischen Kohlenwasserstoffrest darstellt, der gegebenenfalls durch 1 oder 2 Sauerstoffatome in der Kette unterbrochen ist und/oder der durch eine gegebenenfall durch Halogen, Cyano, Dialkylamino, Alkoxy, Alkyl, Trifluormethyl oder Nitro substituierte Phenyl-, Phenoxy-, Phenylthio- oder Phenylsulfonylgruppe oder durch eine α-, β- oder γ-Pyridiylgruppe oder durch eine Aminogruppe substituiert sein kann, wobei diese Aminogruppe zwei gleiche oder verschiedene Substituenten aus der Gruppe Alkyl, Alkoxyalkyl, Aryl und Aralkyl trägt und wobei diese Substituenten gegebenenfalls mit dem Stickstoffatom einen 5- bis 7-gliedrigen Ring bilden, der als weiteres Heteroatom ein Sauerstoff-

BAD ORIGINAL

oder Schwefelatom oder die N-Alkyl-Gruppierung enthalten kann,
oder in der $R^6$
für einen Arylrest steht, der gegebenenfalls 1 bis 3 gleiche oder verschiedene Substituenten aus der Gruppe Alkyl, Alkoxy, Halogen, Cyano, Trifluormethyl, Trifluormethoxy, Dialkylamino oder Nitro enthält.

Es wurde gefunden, daß man die 1,4-Dihydropyridin-Derivate der Formel (I) erhält, wenn man

A) Ylidenverbindungen der Formel II,

$$R\text{-}CH{=}C{<}^{CO\text{-}R^1}_{X} \qquad (II)$$

in welcher
R, $R^1$ und X die oben angegebene Bedeutung haben,
mit Enaminocarbonsäureestern der Formel III,

$$R^3\text{-}\underset{R^2NH}{C}{=}CH\text{-}COO\text{-}Y\text{-}S(O)_n\text{-}Z \qquad (III)$$

in welcher
$R^2$, $R^3$, Y, Z und n die oben angegebene Bedeutung haben,
gegebenenfalls in Gegenwart von inerten organischen Lösungsmitteln umsetzt,
oder

B) Ylidenverbindungen der Formel II,

$$R\text{-}CH{=}C{<}^{CO\text{-}R^1}_{X} \qquad (II)$$

in welcher
R, $R^1$ und X die oben angegebene Bedeutung haben,
mit Aminen der Formel IV und β-Ketocarbonsäureestern der Formel V,

$R^2-NH_2$ $R^3-CO-CH_2-COO-Y-S(O)_n-Z$

(IV) (V)

in welcher
$R^2$, $R^3$, Y, Z und n die oben angegebene Bedeutung haben, gegebenenfalls in Gegenwart inerter organischer Lösungsmittel umsetzt,
oder

C) Yliden-β-dicarbonylverbindungen der Formel VI,

$$R-CH=C\begin{matrix} CO-R^3 \\ COO-Y-S(O)_n-Z \end{matrix} \quad (VI)$$

in welcher
R, $R^3$, Y, Z und n die oben angegebene Bedeutung haben, mit Enaminoverbindungen der Formel VII,

$$\begin{matrix} R^1-C=CH-X \\ R^2-NH \end{matrix} \quad (VII)$$

in welcher
$R^1$, $R^2$ und X die oben angegebene Bedeutung haben, gegebenenfalls in Gegenwart inerter organischer Lösungsmittel zu Reaktion bringt,
oder

D) Yliden-β-dicarbonylverbindungen der Formel VI,

$$R-CH=C\begin{matrix} CO-R^3 \\ COO-Y-S(O)_n-Z \end{matrix} \quad (VI)$$

in welcher
R, $R^3$, Y, Z und n die oben angegebene Bedeutung haben, mit Aminen der Formel IV und Keto-Derivaten der Formel VIII,

$R^2-NH_2$ (IV)   $R^1-CO-CH_2-X$ (VIII)

in welchen
$R^2$, $R^1$ und X die oben angegebene Bedeutung haben, gegebenenfalls in Gegenwart inerter organischer Lösungsmittel umsetzt,
oder

E) Aldehyde der Formel IX,

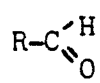 (IX)

in welcher
R die oben angegebene Bedeutung hat, mit Enaminoverbindungen der Formel VII,

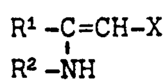 (VII)

in welcher
$R^1$, $R^2$ und X die oben angegebene Bedeutung haben, und β-Ketocarbonsäureestern der Formel V,

$R^3-CO-CH_2-COO-Y-S(O)_n-Z$ (V)

in welcher

$R^3$, Y, Z und n die oben angegebene Bedeutung haben, gegebenenfalls in Gegenwart inerter organischer Lösungsmittel umsetzt,
oder

F) Aldehyde der Formel IX,

$$R\text{-}C{\lesssim}^{H}_{O} \qquad (IX)$$

in welcher
R die oben angegebene Bedeutung hat,
mit Enaminocarbonsäureester der Formel III,

$$\underset{R^2NH}{R^3\text{-}\overset{}{C}}\text{=CH-COO-Y-S(O)}_n\text{-Z} \qquad (III)$$

in welcher
$R^2$, $R^3$, Y, Z und n die oben angegebene Bedeutung haben,
und Keto-Derivaten der Formel VIII,

$$R^1\text{-CO-CH}_2\text{-X} \qquad (VIII)$$

in welcher
$R^1$ und X die oben angegebene Bedeutung haben,
gegebenenfalls in Gegenwart inerter organischer Lösungsmittel

bei Temperaturen zwischen 20°C und 150°C umsetzt.

Die erfindungsgemäßen 1,4-Dihydropyridin-Derivate besitzen wertvolle pharmakologische Eigenschaften. Aufgrund ihrer kreislaufbeeinflussenden Wirkung können sie als antihypertensive

Mittel, als Vasodilatatoren sowie als Coronartherapeutika Verwendung finden und sind somit als Bereicherung der Pharmazie anzusehen.

Je nach Art der verwendeten Ausgangsstoffe kann die Synthese der erfindungsgemäßen Verbindungen durch folgende Formelschemata wiedergegeben werden, wobei 1,4-Dihydro-2,6-dimethyl-4-(3'-nitrophenyl)-pyridin-3,5-dicarbonsäure-methyl-(2-äthylthioäthyl)-ester und 1,4-Dihydro-2,6-dimethyl-4-(2'-trifluormethylphenyl)-pyridin-3,5-dicarbonsäure-(2-benzylthioäthyl)-isopropyl-ester als Beispiele gewählt seien :

A)

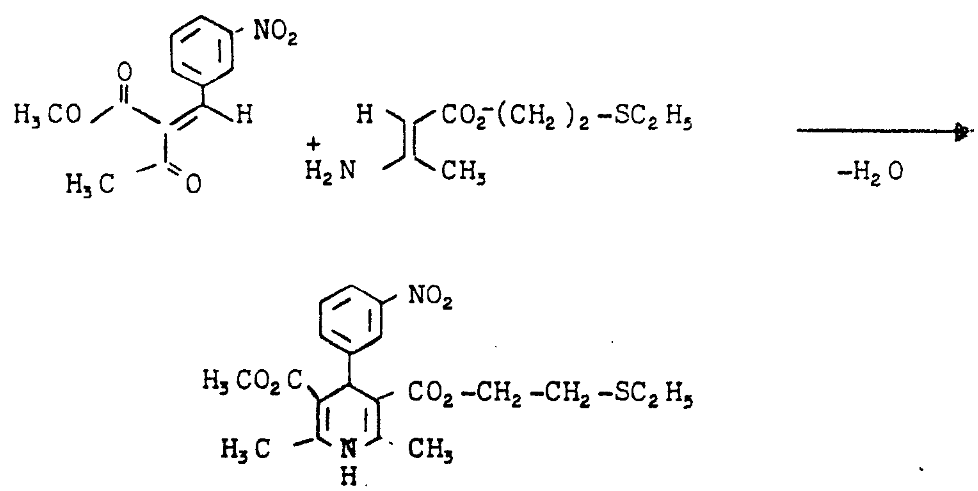


BAD ORIGINAL

B)

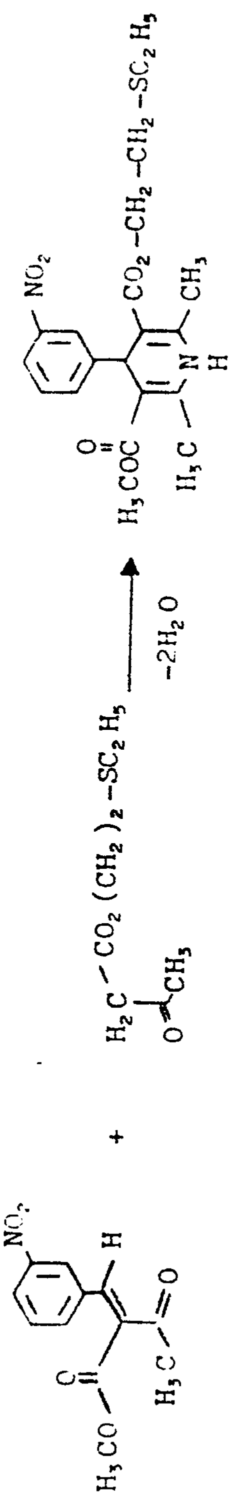


C)

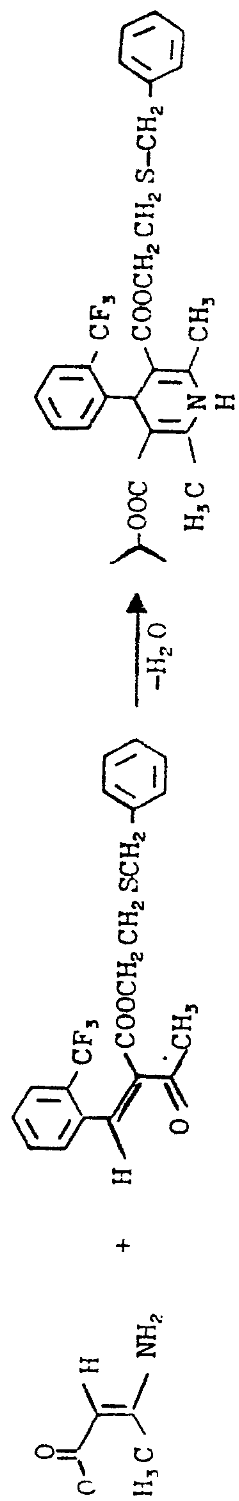


D)

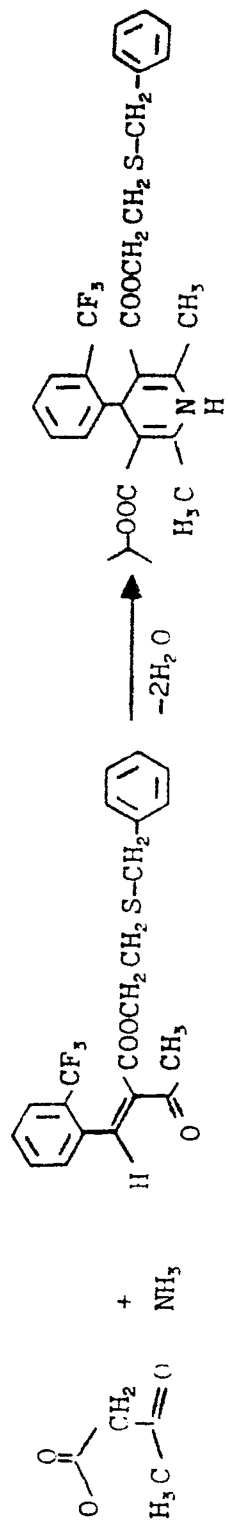


E)

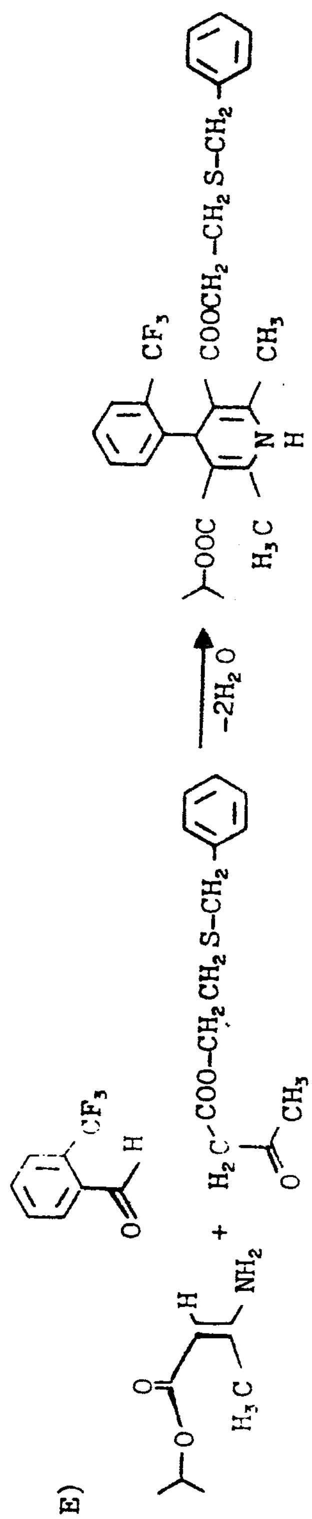

F)

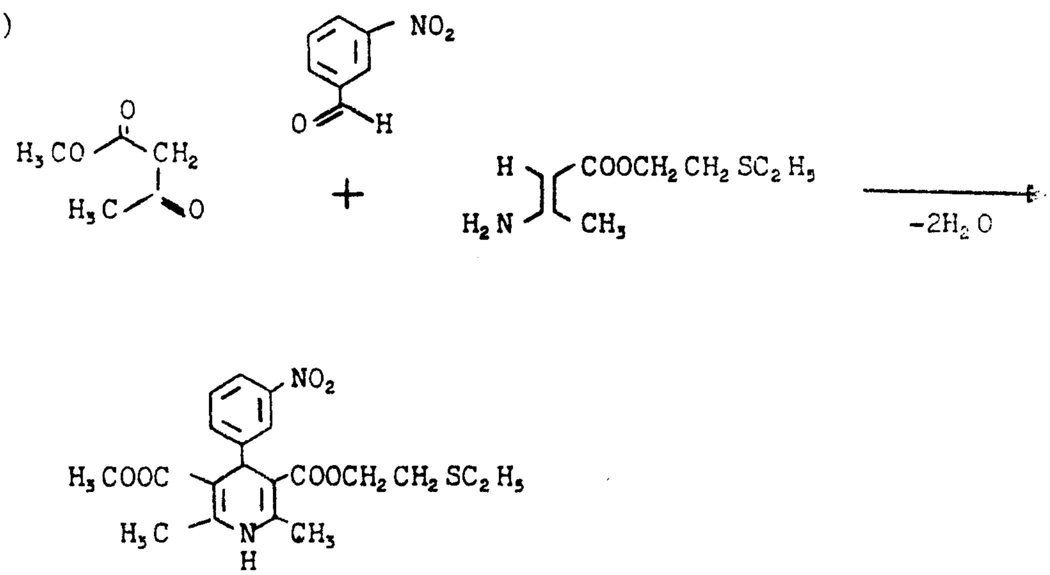


## Verfahrensvariante A

Gemäß Verfahren A wird eine Ylidenverbindung der Formel II

$$R-CH=C\genfrac{}{}{0pt}{}{CO-R^1}{X} \qquad \text{(II)}$$

mit einem Enaminocarbonsäureester der Formel III

$$R^3-\underset{R^2NH}{C}=CH-COO-Y-S(O)_n-Z \qquad \text{(III)}$$

zu Reaktion gebracht.

In der Formel II steht

BAD ORIGINAL



R vorzugsweise für einen Phenyl- oder Naphthylrest oder für einen Thienyl-, Furyl-, Pyrryl-, Pyrazolyl-, Imidazolyl-, Oxazolyl-, Isoxazolyl-, Thiazolyl-, Pyridyl-, Pyridazinyl-, Pyrimidyl-, Pyrazinyl-, Chinolyl-, Isochinolyl-, Indolyl-, Benzimidazolyl-, Chinazolyl- oder Chinoxalylrest. Die genannten Heterocyclen sowie insbesondere der Phenylrest können 1 oder 2 gleiche oder verschiedene Substituenten aufweisen, wobei als Substituenten vorzugsweise Phenyl, geradkettiges oder verzweigtes Alkyl mit 1 bis 8, insbesondere 1 bis 4 Kohlenstoffatomen, Cycloalkyl mit 3 bis 7 Kohlenstoffatomen, Alkenyl oder Alkinyl mit 2 bis 6 Kohlenstoffatomen, insbesondere 2 bis 3 Kohlenstoffatomen, Alkoxy mit vorzugsweise 1 bis 4, insbesondere 1 bis 2 Kohlenstoffatomen, Alkenoxy und Alkinoxy mit 2 bis 6, insbesondere 3 bis 5 Kohlenstoffatomen, Tri-, Tetra- und Pentamethylen, Dioxymethylen,
Halogen wie Fluor, Chlor, Brom oder Jod, insbesondere Fluor, Chlor oder Brom, Trifluormethyl, Trifluormethoxy, Nitro, Cyano, Azido, Hydroxy, Amino, Mono- und Dialkylamino mit vorzugsweise 1 bis 4, insbesondere 1 oder 2 Kohlenstoffatomen je Alkylgruppe, Carboxy, Carbalkoxy mit vorzugsweise 2 bis 4, insbesondere 2 oder 3 Kohlenstoffatomen, Carbonamido, Sulfonamido oder $SO_m$-Alkyl, worin m eine Zahl von 0 bis 2 bedeutet und Alkyl vorzugsweise 1 bis 4, insbesondere 1 oder 2 Kohlenstoffatome enthält, ausgeführt seien.

Weiterhin steht in Formel II

$R^1$ vorzugsweise für Wasserstoff, einen geradkettigen oder verzweigten Alkylrest mit 1 bis 4, insbesondere 1 bis 2 Kohlenstoffatomen, einen Phenylrest oder einen Aralkylrest, insbesondere einen Benzylrest,

X vorzugsweise für die Gruppe $-CO-R^4$, wobei $R^4$ vorzugsweise für einen geradkettigen oder verzweigten Alkylrest mit 1 bis 4 Kohlenstoffatomen, einen Phenylrest, einen Benzylrest, eine Amino-, Monoalkyl- oder eine Dialkylaminogruppe mit bis zu 4 Kohlenstoffatomen je Alkylgruppe steht, wobei die Alkylgruppen gegebenenfalls mit dem Stickstoffatom einen 5- bis 7-gliedrigen Ring bilden, der als weiteres Heteroatom ein Sauerstoff- oder Schwefelatom enthalten kann,
oder
vorzugsweise für die Gruppe $-COOR^5$, wobei $R^5$ vorzugsweise einen geradkettigen, verzweigten oder cyclischen, gesättigten oder ungesättigten Kohlenwasserstoffrest mit bis zu 8, insbesondere mit bis zu 6 Kohlenstoffatomen darstellt, der gegebenenfalls durch 1 oder 2 Sauerstoffatome in der Kette unterbrochen ist und/oder in dem ein Wasserstoffatom durch eine gegebenenfalls durch Halogen, insbesondere Fluor, Chlor oder Brom, Cyano, Dialkylamino mit jeweils 1 bis 2 Kohlenstoffatomen je Alkylgruppe, Alkoxy mit 1 bis 4 Kohlenstoffatomen, Alkyl mit 1 bis 4 Kohlenstoffatomen, Trifluormethyl oder Nitro substituierte

BAD ORIGINAL



Phenyl-,Phenoxy-,Phenylthio- oder Phenylsulfonylgruppe, oder durch eine α-, β- oder γ-Pyridylgruppe oder durch eine Aminogruppe substituiert sein kann, wobei diese Aminogruppe zwei gleiche oder verschiedene Substituenten aus der Gruppe Alkyl mit bis zu 4 Kohlenstoffatomen, Alkoxyalkyl mit bis zu 4 Kohlenstoffatomen, Phenyl und Aralkyl, insbesondere Benzyl, trägt und wobei diese Substituenten gegebenenfalls mit dem Stickstoffatom einen 5- bis 7-gliedrigen Ring bilden, der als weiteres Heteroatom ein Sauerstoff- oder Schwefelatom enthalten kann,

oder

vorzugsweise für die Gruppe $-COO-Y'-S(O)_{n'}-Z'$, wobei diese Gruppe mit dem unten definierten Rest $-COO-Y-S(O)_n-Z$ gleich oder verschieden sein kann, und wobei die Definitionen von Y und Y', Z und Z' und n und n' einander entsprechen,

oder

vorzugsweise für die Gruppe $-S(O)_r-R^6$, in der r = 0, 1 oder 2 bedeutet und $R^6$ vorzugsweise einen geradkettigen, verzweigten oder cyclischen, gesättigten oder ungesättigten aliphatischen Kohlenwasserstoffrest mit bis zu 8, insbesondere bis zu 6 Kohlenstoffatomen darstellt, der gegebenenfalls durch 1 oder 2 Sauerstoffatome in der Kette unterbrochen ist und/oder in dem ein Wasserstoffatom durch eine gegebenenfalls durch Halogen, insbesondere Fluor, Chlor oder Brom, Cyano, Dialkylamino mit jeweils 1 bis 2 Kohlenstoffatomen je Alkylgruppe, Alkoxy mit 1 bis 4 Kohlenstoffatomen, Alkyl mit 1 bis 4 Kohlenstoffatomen, Trifluormethyl oder Nitro substitu-

BAD ORIGINAL



ierte Phenyl-, Phenoxy-, Phenylthio- oder Phenylsulfonylgruppe, oder durch eine α-, ß- oder γ-Pyridylgruppe oder durch eine Aminogruppe substituiert sein kann, wobei diese Aminogruppe zwei gleiche oder verschiedene Substituenten aus der Gruppe Alkyl mit bis zu 4 Kohlenstoffatomen, Alkoxyalkyl mit bis zu 6, insbesondere mit bis zu 4 Kohlenstoffatomen, Phenyl und Aralkyl, insbesondere Benzyl, trägt und wobei diese Substituenten gegebenenfalls mit dem Stickstoffatom einen 5- bis 7-gliedrigen Ring bilden, der als weiteres Heteroatom ein Sauerstoff- oder Schwefelatom enthalten kann,

oder in der $R^6$ für einen Arylrest, insbesondere einen Phenylrest steht, der gegebenenfalls 1, 2 oder 3 gleiche oder verschiedene Substituenten tragen kann, wobei als Substituenten geradkettiges oder verzweigtes Alkyl mit 1 bis 4 Kohlenstoffatomen, Alkoxy mit 1 bis 2 Kohlenstoffatomen, Halogen, insbesondere Fluor, Chlor oder Brom, Cyano, Trifluormethyl, Trifluormethoxy, Dialkylamino mit jeweils 1 bis 2 Kohlenstoffatomen je Alkylgruppe oder Nitro genannt seien.

Die als Ausgangsstoffe verwendeten Ylidenverbindungen der Formel II sind literaturbekannt oder können nach literaturbekannten Methoden hergestellt werden (vgl. z.B. G. Jones "The Knoevenagel Condensation" in Org. Reactions, Vol, XV, 204 ff (1967) und G.Beck und D.Günther, Chem. Ber. 106, 2758 (1973)).

Als Beispiele seien genannt:

BAD ORIGINAL



Benzylidenacetylaceton, ß.ß-Dibenzoylstyrol, 2'-Nitrobenzylidenacetylaceton, 2'-Nitrobenzylidenacetessigsäuremethylester, 3'-Nitrobenzylidenacetessigsäureäthylester, 2'-Trifluormethylbenzylidenacetessigsäure-n-butylester, 2'-Cyanobenzylidenacetessigsäureisopropylester, 3'-Cyanobenzylidenacetessigsäurecyclopentylester, 2'-Methylbenzylidenacetessigsäurealkylester, 2'-Methoxybenzylidenacetessigsäurepropargylester, 2'-Propargyloxybenzylidenacetessigsäuremethylester, 2'-Cyclopropylmethyloxybenzylidenacetessigsäuremethylester, 2'-Chlorbenzylidenacetessigsäure-(2-methoxyäthyl)-ester, 2'-Chlorbenzylidenacetessigsäure-(2-methylthioäthyl)-ester, 3'-Chlorbenzylidenacetessigsäure-(2-dimethylaminoäthyl)-ester, 2'-Brombenzylidenacetessigsäure-(2-(piperidino-1)-äthyl)-ester, 2'-Fluorbenzylidenacetessigsäure-(2-(N-benzyl-N-methylamino)-äthyl)-ester, 3'-Trifluormethoxybenzylidenacetessigsäurepropylester, 2'-Äthinylbenzylidenacetessigsäuremethylester, 3'-Azidobenzylidenacetessigsäureäthylester, 4'-Methoxycarbonylbenzylidenacetessigsäure-n-butylester, 3'-Methylsulfonylbenzylidenacetessigsäureisopropylester, 3'-Methylsulfonylbenzylidenacetessigsäurecyclohexylester, 2'-Nitrobenzylidenacetessigsäureisobutylester, 3'-Nitrobenzylidenacetessigsäure-(2-phenylthioäthyl)-ester, 3'-Chlor-4'-nitrobenzylidenacetessigsäurebenzylester, 4'-Chlor-3'-sulfamoylbenzylidenacetessigsäure-(4-chlorbenzyl)-ester, 3',4'-Dichlorbenzylidenacetessigsäure-(4-trifluormethylbenzyl)-ester, 3'-Cyanobenzylidenacetessigsäure-(2-phenoxyäthyl)-ester, 2'-Nitrobenzylidenacetessigsäure-(2-(pyridyl-2)-äthyl)-ester, 3'-Nitrobenzylidenacetessigsäureamid, 2'-Trifluormethylbenzylidenacetessigsäuredimethylamid, 2'-Nitrobenzylidenpropionylessigsäuremethylester, 2'-Cyanobenzylidenpropionyl-

BAD ORIGINAL



essigsäureäthylester, 2'-Trifluormethylbenzylidenbenzoyl-essigsäuremethylester, 3'-Azidobenzyliden-γ-phenylacet-essigsäuremethylester, α-Acetyl-β-(pyridyl-3)-acrylsäuremethylester, α-Acetyl-β-(chinolinyl-4)-acrylsäure-(2-n-propoxyäthyl)-ester, α-Acetyl-β-(thienyl-2)-acrylsäure-äthylester, α-Acetyl-β-(furyl-2)-acrylsäureisobutyl-ester.

1-Phenyl-2-methylsulfonyl-buten-1-on-3,
1-(2'-Nitrophenyl)-2-methylsulfonyl-buten-1-on-3,
1-(3'-Nitrophenyl)-2-äthylsulfonyl-buten-1-on-3,
1-(2'-Trifluormethylphenyl)-2-methylsulfonyl-buten-1-on-3,
1-(2'-Cyanophenyl)-2-methylsulfonyl-buten-1-on-3,
1-(2'-Methylphenyl)-2-methylsulfonyl-buten-1-on-3,
1-(2'-Methoxyphenyl)-2-methylsulfonyl-buten-1-on-3,
1-(2'-Chlorphenyl)-2-methylsulfonyl-buten-1-on-3,
1-(3'-Cyanophenyl)-2-n-butylsulfonyl-buten-1-on-3,
1-(2'-Nitrophenyl)-2-(2-methoxyäthylsulfonyl)-buten-1-on-3,
1-(3'-Nitrophenyl)-2-cyclopentylsulfonyl-buten-1-on-3,
1-(2'-Trifluormethylphenyl)-2-(2-dimethylaminoäthylsulfonyl)-buten-1-on-3
1-(2'-Cyanophenyl-2-(2-(piperidino-1)-äthylsulfonyl)-buten-1-on-3,
1-(2'-Nitrophenyl)-2-(2-(N-benzyl-N-methylamino)-äthylsulfonyl)-buten-1-on-3,
1-(3'-Nitrophenyl)-2-benzylsulfonyl-buten-1-on-3,
1-(3'-Cyanophenyl)-2-(2-phenoxyäthylsulfonyl)-buten-1-on-3,
1-(3'-Nitrophenyl)-2-(2-(pyridyl-2)-äthylsulfonyl)-buten-1-on-3,
1-(2'-Trifluormethylphenyl)-2-phenylsulfonyl-buten-1-on-3,
1-(2'-Nitrophenyl)-2-(3-chlorphenylsulfonyl)-buten-1-on-3,
1-(3'-Nitrophenyl-2-(4-methylphenylsulfonyl)-buten-1-on-3,
1-(3'-Nitrophenyl-2-(4-methoxyphenylsulfonyl)-buten-1-on-3.

BAD ORIGINAL



1-(2'-Trifluormethylphenyl)-2-(4-nitrophenylsulfonyl)-buten-1-on-3,
1-(2'-Nitrophenyl-2-(4-trifluormethylphenylsulfonyl)-buten-1-on-3,
1-(2'-Nitrophenyl)-2-methylsulfonyl-penten-1-on-3,
1-(3'-Nitrophenyl)-2-methylsulfonyl-4-phenyl-buten-1-on-3,
1-(2'-Nitrophenyl)-2-phenylsulfonyl-3-phenylpropen-1-on-3.

1-(Pyridyl-3)-2-methylsulfonyl-buten-1-on-3,
1-(Pyridyl-2)-2-n-butylsulfonyl-buten-1-on-3,
1-(Pyridyl-3)-2-phenylsulfonyl-buten-1-on-3,
1-(Chinolinyl-4)-2-methylsulfonyl-buten-1-on-3,
1-(Thienyl-2)-2-(4-trifluormethylphenylsulfonyl)-buten-1-on-3,
1-(Furyl-2)-2-(3,4-dichlorphenylsulfonyl)-buten-1-on-3.

In der Formel III stehen

$R^2$ vorzugsweise für ein Wasserstoffatom oder für einen geradkettigen oder verzweigten Alkylrest mit 1 bis 8 Kohlenstoffatomen, insbesondere 1 bis 4 Kohlenstoffatomen, wobei der Alkylrest gegebenenfalls durch ein Sauerstoffatom in der Alkylkette unterbrochen ist, oder für einen Phenylrest oder für einen Aralkylrest, insbesondere für einen Benzylrest,

$R^3$ für Wasserstoff, einen geradkettigen oder verzweigten Alkylrest mit 1 bis 4, insbesondere mit 1 bis 2 Kohlenstoffatomen, einen Phenylrest oder einen Aralkylrest, insbesondere einen Benzylrest,

Y vorzugsweise für eine $-(CH_2)_s$-Gruppe (s = 2 bis 5), in der gegebenenfalls ein Wasserstoffatom durch eine niedere Alkylgruppe substituiert ist,

n für 0, 1 oder 2 und

Z vorzugsweise für einen geradkettigen, verzweigten oder cyclischen, gesättigten oder ungesättigten aliphatischen Kohlenwasserstoffrest mit bis zu 8, insbesondere mit bis zu 6 Kohlenstoffatomen, der gegebenenfalls durch 1 oder 2 Sauerstoffatome in der Kette oder im Ring unterbrochen ist und/oder in dem ein Wasserstoffatom durch eine gegebenenfalls durch Halogen, insbesondere Fluor, Chlor oder Brom, Cyano, Dialkylamino mit jeweils 1 bis 2 Kohlenstoffen je Alkylgruppe, Alkyl mit 1 bis 4 Kohlenstoffatomen, Alkoxy mit 1 bis 4 Kohlenstoffatomen, Trifluormethyl oder Nitro substituierte Phenoxy- oder Phenylgruppe oder durch eine α-, β- oder γ-Pyridylgruppe substituiert sein kann.

Die als Ausgangsstoffe verwendeten Enaminocarbonsäureester der Formel III können nach literaturbekannten Methoden hergestellt werden (vgl. A.C.Cope, J.Amer.chem.Soc. 67, 1017 (1945) ).

Als Beispiele seien genannt :

β-Amino-crotonsäure-(2-methylthioäthyl)-ester,
β-Methylamino-crotonsäure-(2-methylthioäthyl)-ester,
β-n-Butylamino-crotonsäure-(2-methylthioäthyl)-ester,
β-iso-Butylamino-crotonsäure(2-äthylthioäthyl)-ester,
β-(2-Methoxyäthylamino)-crotonsäure-(2-äthylthioäthyl)-ester,
β-Anilino-crotonsäure-(2-äthylthioäthyl)-ester,
β-Benzylamino-crotonsäure-(2-äthylthioäthyl)-ester,
β-Amino-β-propyl-acrylsäure-(2-n-butylthioäthyl)-ester,
β-Amino-zimtsäure-(2-iso-butylthioäthyl)-ester,
β-Amino-β-benzyl-acrylsäure-(2-äthylthioäthyl)-ester,
β-Amino-crotonsäure(2-methylsulfonyläthyl)-ester,

BAD ORIGINAL



β-Amino-crotonsäure-(2-cyclohexylthioäthyl)-ester,
β-Amino-crotonsäure-(2-(2-methoxyäthylthio)-äthyl)-ester,
β-Amino-cortonsäure-(2-benzylthioäthyl)-ester,
β-Amino-crotonsäure-(2-(2-benzyl-äthylthio)-äthyl)-ester,
β-Amino-crotonsäure-(2-(2-phenoxyäthylthio)-äthyl-ester,
β-Amino-cortonsäure-(2-(pyridyl-2-methylthio)-ätheyl)-ester,
β-Amino-crotonsäure-(2-(pyridyl-3-methylthio)-äthyl)-ester,
β-Amino-cortonsäure-(2-(4-chlor-benzylthio)-äthyl)-ester,
β-Amino-crotonsäure-(2-(3-cyanobenzylthio)-äthyl)-ester,
β-Amino-crotonsäure-(2-(4-methylbenzylthio)-äthyl)-ester,
β-Amino-crotonsäure-(2-(4-dimethylaminobenzylthio)-äthyl)-ester,
β-Amino-cortonsäure-(2-(3-methylbenzylthio)-äthyl)-ester,
β-Amino-crotonsäure-(2-(4-methoxybenzylthio)-äthyl)-ester,
β-Amino-crotonsäure-(2-(4-trifluormethylbenzylthio)-äthyl)-ester,
β-Amino-crotonsäure-(2-(4-nitrobenzylthio)-äthyl)-ester.

Als Verdünnungsmittel kommen alle inerten organischen Lösungsmittel in Frage. Hierzu gehören vorzugsweise Alkohole wie Aethanol, Methanol, Isopropanol, Aether wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20°C und 150°C, vorzugsweise zwischen 20°C und 100°C, insbesondere bei der Siedetemperatur des jeweiligen Lösungsmittel.

Die Umsetzung kann bei Normaldruck, aber auch bei erhöhtem Druck durchgeführt werden. Im allgemeinen arbeitet man unter Normaldruck.

BAD ORIGINAL



Bei der Durchführung des erfindungsgemäßen Verfahrens wird ein Mol der Ylidenverbindung der Formel II mit einem Mol Enaminocarbonsäureester der Formel III in einem geeigneten Lösungsmittel zur Reaktion gebracht. Die Isolierung und Reinigung der erfindungsgemäßen Substanzen erfolgt vorzugsweise derart, daß man das Lösungsmittel im Vakuum abdestilliert und den gegebenenfalls erst nach Eiskühlung kristallin erhaltenen Rückstand aus einem geeigneten Lösungsmittel umkristallisiert.

Verfahrensvariante B

Gemäß Verfahren B wird eine Ylidenverbindung der Formel II

$$R\text{-}CH{=}C\begin{matrix}\diagup CO\text{-}R^1 \\ \diagdown X\end{matrix} \qquad (II)$$

mit einem Amin der Formel IV und einem β-Ketocarbonsäureester der Formel V

$R^2\text{-}NH_2$ (IV)

$R^3\text{-}CO\text{-}CH_2\text{-}COO\text{-}Y\text{-}S(O)_n\text{-}Z$ (V)

zur Reaktion gebracht.

BAD ORIGINAL

In den Formeln II, IV und V haben die Reste R, $R^1$, $R^2$, $R^3$, X, Y, Z und n vorzugsweise die unter Verfahrensvariante A angegebene Bedeutung.

Beispiele der als Ausgangssubstanzen verwendeten Ylidenverbindungen der Formel II sind bereits unter Verfahrensvariante A aufgeführt.

Die erfindungsgemäß verwendbaren Amine der Formel IV sind bereits bekannt.

Als Beispiele seien genannt :

Ammoniak, Methylamin, n-Propylamin, Isopropylamin, n-Butylamin, Isobutylamin, β-Methoxyäthylamin, Benzylamin, Anilin.

Die als Ausgangsstoffe verwendeten β-Ketocarbonsäureester der Formel V können nach literaturbekannten Methoden hergestellt werden (z.B. D.Borrmann, "Umsetzung von Diketen mit Alkoholen, Phenolen und Mercaptanen", in Houben-Weyl, Methoden der Organischen Chemie Vol. VII/4, 230ff (1968)).

Als Beispiele seien genannt :

Acetessigsäure-(2-methylthioäthyl)-ester;
Propionylessigsäure-(2-methylthioäthyl)-ester,
γ-Phenylacetessigsäure-(2-äthylthioäthyl)-ester,
Benzoylessigsäure-(2-äthylthioäthyl)-ester,
Acetessigsäure-(2-n-butylthioäthyl)-ester,
Acetessigsäure-(2-iso-butylthioäthyl)-ester,
Acetessigsäure-(2-methylsulfonyläthyl)-ester,
Acetessigsäure-(2-cyclohexylthioäthyl)-ester,

BAD ORIGINAL

Acetessigsäure-(2-(2-methoxyäthylthio)-äthyl)-ester,
Acetessigsäure-(2-benzylthioäthyl)-ester,
Acetessigsäure-(2-benzyltäthylthio)-äthyl)-ester,
Acetessigsäure-(2-(2-phenoxyäthylthio)-äthyl)-ester,
Acetessigsäure-(2-(pyridyl-2-methylthio)-äthyl)-ester,
Acetessigsäure-(2-(pyridyl-3-methylthio)-äthyl)-ester,
Acetessigsäure-(2-(4-chlorbenzylthio)-äthyl)-ester,
Acetessigsäure-(2-(4-methylbenzylthio)-äthyl)-ester,
Acetessigsäure-(2-(4-dimethylaminobenzylthio)-äthyl)-ester,
Acetessigsäure-(2-(4-methoxybenzylthio)-äthyl)-ester,
Acetessigsäure-(2-(4-trifluormethylbenzylthio)-äthyl)-ester,
Acetessigsäure-(2-(4-nitrobenzylthio)-äthyl)-ester.

Als Verdünnungsmittel kommen alle inerten organischen Lösungsmittel in Frage. Hierzu gehören vorzugsweise Alkohole wie Aethanol, Methanol, Isopropanol, Aether wie Dioxan, Diäthyläther, Tetrahydrofuran, Glykolmonomethyläther, Glykoldimethyläther oder Eisessig, Dimethylformamid, Dimethylsulfoxi Acetonitril, Pyridin und Hexamethylphosphorsäuretriamid.

Die Reaktionstemperaturen können in einem größeren Bereich variiert werden. Im allgemeinen arbeitet man zwischen 20°C und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formel II, IV und V jeweils in molaren Mengen eingesetzt. Das verwendete Amin wird zweckmäßigerweise im Ueberschuß von 1 bis 2 Mol zugegeben. Die erfindungsgemäßen Verbindungen können leicht durch Umkristallisation aus einem geeigneten Lösungsmittel gereinigt werden.

Verfahrensvariante C

Gemäß Verfahren C wird eine Yliden-β-dicarbonylverbindung der Formel VI

$$R{-}CH{=}C\genfrac{}{}{0pt}{}{-CO-R^3}{-COO-Y-S(O)_n-Z} \qquad (VI)$$

mit einer Enaminverbindung der Formel VII

$$\genfrac{}{}{0pt}{}{R^1-C=CH-X}{R^2NH\quad} \qquad (VII)$$

zur Reaktion gebracht.

In den Formeln VI und VII haben R, $R^1$, $R^2$, $R^3$, X, Y, Z und n vorzugsweise die unter Verfahrensvariante A angegebene Bedeutung.

Die als Ausgangsstoffe verwendeten Yliden-β-dicarbonylverbindungen der Formel VI können nach literaturbekannten Methoden hergestellt werden (vgl. z.B. G.Jones, "The Knoevenagel Condendation" in Org. Reactions, Vol. XV, 204ff (1967)).

BAD ORIGINAL

Als Beispiele seien genannt :

2'-Nitrobenzylidenacetessigsäure-(2-methylthioäthyl)-ester,
3'-Nitrobenzylidenpropionylessigsäure-(2-methylthioäthyl)-ester,
2'-Methoxybenzylidenbenzoylessigsäure-(2-äthylthioäthyl)-ester,
3'-Methylbenzylidenacetessigsäure-(2-n-butylthioäthyl)-ester,
2'-Chlorbenzylidenacetessigsäure-(2-iso-butylthioäthyl)-ester,
3'-Brombenzylidenacetessigsäure-(2-methylsulfonyläthyl)-ester,
3'-Cyanobenzylidenacetessigsäure-(2-cyclopentylthioäthyl)-ester,
2'-Trifluormethylbenzylidenacetessigsäure-(2-(2-methoxyäthyl-thio)-äthyl)-ester,
3'-Trifluormethylbenzylidenacetessigsäure-(2-benzylthioäthyl)-ester,
3'-Trifluormethoxybenzylidenacetessigsäure-(2-(2-phenoxy-äthylthio)-äthyl)-ester,
3'-4'-Dioxymethylenbenzylidenacetessigsäure-(2-(pyridyl-2-methylthio)-äthyl)-ester,
2'-Propargylbenzylidenacetessigsäure-(2-(4-chlorbenzylthio)-äthyl)-ester,
2'-Allylbenzylidenacetessigsäure-(2-(4-methylbenzylthio)-äthyl)-ester,
2'-Propargyloxybenzylidenacetessigsäure-(2-(4-methoxybenzyl-thio)-äthyl)-ester,
3'-Methylsulfonylbenzylidenacetessigsäure-(2-(4-trifluormethyl-benzylthio)-äthyl)-ester,
3'-4'-Tetramethylenbenzylidenacetessigsäure-(2-(4'-nitro-benzylthio)-äthyl)-ester,
2-Acetyl-3-(pyridyl-3)-acrylsäure-(2-äthylthioäthyl)-ester,

BAD ORIGINAL



2-Acetyl-3-(thienyl-2)-acrylsäure-(2-methylthioäthyl)-ester,
2-Acetyl-3-(furyl-2)-acrylsäure-(3-methylthiopropyl)-ester,
2-Acetyl-3-(chinolinyl-4)-acrylsäure-(2-benzylthioäthyl)-ester,
2-Acetyl-3-(pyridyl-2)-acrylsäure-(2-methylthioäthyl)-ester.

Die als Ausgangsstoffe verwendeten Enaminoverbindungen der Formel VII sind literaturbekannt oder können nach literaturbekannten Methoden hergestellt werden (vgl. A.C.Cope, J. Amer.chem.Soc. 67, 1017 (1945), N.Guruswamy et al. Indian J.Chem. 11, 882 (1973) ).

Als Beispiel seien genannt :

4-Amino-3-penten-2-on,
3-Amino-1.3-diphenyl-acrolein,
4-Amino-1.5-diphenyl-3-penten-2-on,
β-Aminocrotonsäureamid,
β-Aminocrotonsäure-n-butylamid,
β-Aminocrotonsäuredimethylamid,
β-Aminocrotonsäuremethylester,
β-Methylaminocrotonsäure-n-butylester,
β-n-Butylaminocrotonsäureisopropylester,
β-Benzylaminocrotonsäurecyclopentylester,
β-(2-Methoxyäthylamino)-crotonsäureallylester,
β-Anilinocrotonsäurepropargylester,
β-Amino-β-äthylacrylsäure-(2-[illegible]äthyl)-ester,
β-Amino-β-benzyl-acrylsäure-(2-propoxyäthyl)-ester,
β-Amino-crotonsäurebenzylester,
β-Aminocrotonsäure-(2-phenyläthyl)-ester,
β-Aminocrotonsäure-(2-phenoxyäthyl)-ester,
β-Aminocrotonsäure-(2-phenylthioäthyl)-ester,
β-Aminocrotonsäure-(2-phenyl-[illegible]phenyläthyl)-ester,

BAD ORIGINAL



β-Aminocrotonsäure-(2-methylthioäthyl)-ester,
β-Aminocrotonsäure-(2-äthylthioäthyl)-ester,
β-Aminocrotonsäure-(2-methylsulfonyläthyl)-ester,
β-Aminocrotonsäure-(4-chlorbenzyl)-ester,
β-Aminocrotonsäure-(4-methoxybenzyl)-ester,
β-Aminocrotonsäure-(4-methylbenzyl)-ester,
β-Aminocrotonsäure-(4-trifluormethylbenzyl)-ester,

β-Aminocrotonsäure-(3,4-dichlorbenzyl)-ester,
β-Aminocrotonsäure-(pyridyl-2-methyl)-ester,
β-Aminocrotonsäure-(2-dimethylaminoäthyl)-ester,
β-Aminocrotonsäure-(2-(N-benzyl-N-methylamino)-äthyl)-ester,
β-Aminocrotonsäure-(2-(piperidino-1)-äthyl)-ester,
β-Aminocrotonsäure-(2-(morpholino-4)-äthyl)-ester.

2-Amino-1-methylsulfonyl-propen-1,
2-Amino-1-methylsulfonyl-buten-1,
α-Amino-β-methylsulfonylstyrol,
2-Amino-1-äthylsulfonyl-buten-1,
2-Amino-1-isobutylsulfonyl-propen-1,
2-Amino-1-cyclopentylsulfonyl-propen-1,
2-Amino-1-(2-methoxyäthylsulfonyl)-propen-1,
2-Amino-1-(2-propoxyäthylsulfonyl)-propen-1,
2-Amino-1-(benzylsulfonyl)-propen-1,
2-Amino-1-(2-phenoxyäthylsulfonyl)-propen-1,
2-Amino-1-(2-dimethylaminoäthylsulfonyl)-propen-1,
2-Amino-1-(2-N-benzyl-N-methylaminoäthylsulfonyl)-propen-1,
2-Methylamino-1-methylsulfonyl-propen-1,
2-Methylamino-1-äthylsulfonyl-propen-1,
2-Aethylamino-1-benzylsulfonyl-propen-1,

2-Amino-1-phenylsulfonyl-propen-1,
2-Amino-1-(4-chlorphenylsulfonyl)-propen-1,
2-Amino-1-(4-methylphenylsulfonyl)-propen-1,
2-Amino-1-(4-methoxyphenylsulfonyl)-propen-1,
2-Amino-1-(4-trifluormethylphenylsulfonyl)-propen-1,
2-Amino-1-(4-nitrophenylsulfonyl)-propen-1,
2-Amino-1-(4-trifluormethoxyphenylsulfonyl)-propen-1,
2-Amino-1-(3,4-dichlorphenylsulfonyl)-propen-1,
2-Amino-1-(4-chlor-3-trifluormethylphenylsulfonyl)-propen-1.

Als Verdünnungsmittel kommen alle inerten organischen Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20°C und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol der Yliden-ß-dicarbonylverbindung der Formel VI mit einem Mol Enaminoverbindung der Formel VII in einem geeigneten Lösungsmittel zur Reaktion gebracht.

BAD ORIGINAL



Verfahrensvariante D

Gemäß Verfahren D wird eine Yliden-β-dicarbonylverbindung der Formel VI

$$R\text{-}CH{=}C\begin{matrix}\diagup CO\text{-}R^3 \\ \diagdown COO\text{-}Y\text{-}S(O)_n\text{-}Z\end{matrix} \qquad (VI)$$

mit einem Amin der Formel IV und einem Keto-Derivat der Formel VIII

$R^2-NH_2$ (IV)

$R^1-CO-CH_2-X$ (VIII)

zur Reaktion gebracht.

In den Formeln VI, IV und VIII haben die Reste R, $R^1$, $R^2$, $R^3$, X, Y, Z und n vorzugsweise die unter Verfahrensvariante A angegebene Bedeutung.

Beispiele der als Ausgangsverbindungen verwendeten Yliden-β-dicarbonylverbindungen der Formel VI sind bereits unter Verfahrensvariante C aufgeführt.

Die erfindungsgemäß verwendbaren Amine der Formel IV sind bereits unter Verfahrensvariante B beschrieben.

Die als Ausgangssubstanzen eingesetzten [illegible] der Formel VIII sind literaturbekannt oder können nach literaturbekannten Methoden hergestellt werden (vgl. z.B. D.Borrmann, "Umsetzung von Diketen mit Alkohlen, Phenolen und Mercaptanen", in Houben-Weyl, Methoden der Organischen Chemie, Vol.VII/4, 230ff (1968); H.O.House und J.K.Larson, J.Org.Chem. 33, 61 (1968)).

BAD ORIGINAL



Als Beispiele seien genannt:

Acetylaceton, Benzoylaceton, ω-Benzylacetophenon, Acetessigsäureamid, Acetessigsäure-n-butyl-amid, Acetessigsäuredimethylamid, Formylessigsäureäthylester, Acetessigsäuremethylester, n-Propionylessigsäureäthylester, Benzoylessigsäureäthylester, Acetessigsäureisopropylester, Acetessigsäurecyclopentylester, Acetessigsäureallylester, Acetessigsäureproparylester, Acetessigsäure-(2-methoxyäthyl)-ester, Acetessigsäure-(2-propoxyäthyl)-ester, Acetessigsäurebenzylester, Acetessigsäure-(2-phenylathylester), Acetessigsäure-(2-phenoxyäthyl)-ester, Acetessigsäure-(2-phenylthioäthyl)-ester, Acetessigsäure-(2-phenylsulfonyläthyl)-ester, Acetessigsäure-(2-methylthioäthyl)-ester, Acetessigsäure-(2-methylsulfonyläthyl)-ester, Acetessigsäure-(2-benzylthioäthyl)-ester, Acetessigsäure-(pyridyl-2-methyl)-ester, Acetessigsäure-(2-dimethylaminoäthyl)-ester, Acetessigsäure-(2-N-benzyl-N-methylamino)-äthyl)-ester, Acetessigsäure-(2-piperidino-1)-äthyl)-ester, Acetessigsäure-(2-(morpholino-4)-äthyl)-ester, Methylsulfonylaceton, n-Butylsulfonylaceton, Isobutylsulfonylaceton, Cyclopentylsulfonylaceton, (2-Methoxyäthylsulfonyl)-aceton, (2-Propoxyäthylsulfonyl)-aceton, Benzylsulfonylaceton, 4-Chlorbenzylsulfonylaceton, 4-Trifluormethylbenzylsulfonylaceton, (2-Phenyläthylsulfonyl)-aceton, (2-Phenoxyäthylsulfonyl)-aceton, (2-Dimethylaminoäthylsulfonyl)-aceton, (2-N-Benzyl-N-methylamino-äthylsulfonyl)-aceton, (2-(Piperidino-1-)-äthylsulfonyl)-aceton, Phenylsulfonylaceton, 4-Chlorphenylsulfonylaceton, 4-Fluorphenylsulfonylaceton, 3,4-Dichlorphenylsulfonylaceton, 4-Trifluormethylsulfonylaceton, 4-Trifluormethoxyphenylsulfonylaceton, 4-Chlor-3-trifluormethylphenylsulfonylaceton, 4-Methylphenylsulfonylaceton, 4-tert.-Butylphenylsulfonylaceton, 4-Methoxyphenylsulfonylaceton, 4-Nitrophenylsulfonylaceton, ω-Methylsulfonylacetophenon.

BAD ORIGINAL



Als Verdünnungsmittel kommen alle inerten organischen Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20°C und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formel VI, IV und VIII jeweils in molaren Mengen eingesetzt. Das verwendete Amin wird zweckmäßigerweise im Überschuß von 1 bis 2 Mol zugegeben.

BAD ORIGINAL



Verfahrensvariante E

Gemäß Verfahren E wird ein Aldehyd der Formel IX

$$R-C\begin{smallmatrix}\nearrow H\\ \searrow O\end{smallmatrix} \qquad (IX)$$

mit einer Enaminoverbindung der Formel VII

$$\begin{matrix}R^1-C=CH-X\\ \quad\;| \\ R^2NH\end{matrix} \qquad (VII)$$

und einem β-Keto-carbonsäureester der Formel V

$$R^3-CO-CH_2-COO-Y-S(O)_n-Z \qquad (V)$$

zur Reaktion gebracht.

In den Formeln IX, VII und V haben R, $R^1$, $R^2$, $R^3$, X, Y, Z und n vorzugsweise die unter Verfahrensvariante A angegebene Bedeutung.

Die als Ausgangsstoffe verwendeten Aldehyde der Formel IX sind literaturbekannt oder können nach literaturbekannten Methoden hergestellt werden (vgl. z.B. E.Mosettig, Org. Reactions VIII, 218ff (1954)).

Als Beispiele seien genannt:

Benzaldehyd, 2-, 3- oder 4-Phenylbenzaldehyd, α- oder β-Naphthylaldehyd, 2-, 3- oder 4-Methylbenzaldehyd, 2- oder 4-n-Butylbenzaldehyd, 2-, 3- oder 4-Isopropylbenzaldehyd, 2- oder 4-Cyclopropylbenzaldehyd, 2-Vinylbenzaldehyd, 2-Äthinylbenzaldehyd, 2,3-Tetramethylenbenzaldehyd, 3,4-Dioxymethylenbenzaldehyd, 2-, 3- oder 4-Methoxybenzaldehyd, 2-Cyclopropylmethyloxybenzaldehyd, 2-Propargyloxybenzaldehyd, 2-Allyloxybenzaldehyd, 2-, 3- oder 4-Chlor/Brom/Fluorbenzaldehyd, 2-, 3- oder 4-Trifluormethylbenzaldehyd, 2-, 3- oder 4-Trifluormethoxybenzaldehyd, 4-Hydroxybenzaldehyd, 2-, 3- oder 4-Nitrobenzaldehyd, 2-, 3- oder 4-Cyanobenzaldehyd, 3-Azidobenzaldehyd, 2-, 3- oder 4-Dimethylaminobenzaldehyd, 3-Carboäthoxybenzaldehyd, 3- oder 4-Carbamoylbenzaldehyd, 2-, 3- oder 4-Methylmercaptobenzaldehyd, 2-, 3- oder 4-Methylsulfinylbenzaldehyd, 2-, 3- oder 4-Methylsulfonylbenzaldehyd, 3,4,5-Trimethoxybenzaldehyd, 2,4- oder 2,6-Dichlorbenzaldehyd, 2,4-Dimethylbenzaldehyd, 2,4- oder 2,6-Dinitrobenzaldehyd, 2-Chlor-6-nitrobenzaldehyd, 4-Chlor-2-nitrobenzaldehyd, 2-Nitro-4-methoxybenzaldehyd, 2-Nitro-4-cyanobenzaldehyd, 2-Chlor-4-cyanobenzaldehyd, 4-Cyano-2-methylbenzaldehyd, 3-Methyl-4-trifluormethylbenzaldehyd, 3-Chlor-4-trifluormethylbenzaldehyd, 4-Chlor-3-sulfamoylbenzaldehyd.

Thiophen-2-aldehyd, Furan-2-aldehyd, Pyrrol-2-aldehyd, Pyrazol-4-aldehyd, Imidazol-2-aldehyd, Oxazol-2-aldehyd, Isoxazol-3-aldehyd, Thiazol-2-aldehyd, Pyridin-2-aldehyd, Pyridin-3-aldehyd, Pyridin-4-aldehyd, 4-Methyl-pyridin-2-aldehyd, 6-Methylpyridin-2-aldehyd, Pyridazin-4-aldehyd, Pyrimidin-4-aldehyd, Pyrazin-2-aldehyd, Chinolin-4-aldehyd, Isochinolin-1-aldehyd, Indol-3-aldehyd, Benzimidazol-2-aldehyd, Chinazolin-2-aldehyd, Chinoxalin-2-aldehyd.

BAD ORIGINAL



Die erfindungsgemäß verwendbaren Enaminoverbindungen der Formel VII sind bereits unter Verfahrensvariante C, die erfindungsgemäß eingesetzten 3-Ketocarbonsäureester der Formel V unter Verfahrensvariante B angegeben.

Als Verdünnungsmittel kommen alle inerten organischen Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20°C und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formel IX, VII und V jeweils etwa in molaren Mengen eingesetzt.

BAD ORIGINAL



Verfahrensvariante F

Gemäß Verfahren F wird ein Aldehyd der Formel IX

$$R-C\begin{matrix} \nearrow H \\ \searrow O \end{matrix} \qquad (IX)$$

mit einem Enaminocarbonsäureester der Formel III

$$R^3-\underset{R^2NH}{\underset{|}{C}}=CH-COO-Y-S(O)_n-Z \qquad (III)$$

und einem Keto-Derivat der Formel VIII

$$R^1-CO-CH_2-X \qquad (VIII)$$

zur Reaktion gebracht.

In den Formel IX, III und VIII haben R, $R^1$, $R^2$, $R^3$, X, Y, Z und n vorzugsweise die unter Verfahrensvariante A angegebene Bedeutung.

Die als Ausgangssubstanzen verwendeten Aldehyde der Formel IX sind unter Verfahrensvariante E, die Enaminocarbonsäureester der Formel III unter Verfahrensvariante A und die Keto-Derivate der Formel VIII unter Verfahrensvariante D aufgeführt.

Als Verdünnungsmittel kommen alle inerten organischen Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

BAD ORIGINAL



Die Reaktionstemperaturen können in einem größeren Bereich variiert werden. Im allgemeinen arbeitet man zwischen 20°C und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formel IX, III und VIII jeweils etwa in molaren Mengen eingesetzt.

Die vorstehenden Herstellungsverfahren sind lediglich zur Verdeutlichung angegeben, und die Herstellung der Verbindungen der Formel I ist nicht auf diese Verfahren beschränkt, sondern jede Modifikation dieser Verfahren ist in gleicher Weise für die Herstellung der erfindungsgemäßen Verbindungen anwendbar.

Je nach der Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z.B. E.L. Eliel, Stereochemistry of Carbon Compounds, McGraw Hill, 1962).

Außer den unten angeführten Herstellungsbeispielen seien folgende erfindungsgemäßen Wirkstoffe genannt:

BAD ORIGINAL



1.4-Dihydro-2.6-dimethyl-4-(2'-nitrophenyl)-pyridin-3.5-dicarbonsäure-methyl-(2-methylthioäthyl)-ester,
1.4-Dihydro-2.6-dimethyl-4-(2'-nitrophenyl)-pyridin-3.5-dicarbonsäure-n-butyl-(2-methylthioäthyl)-ester,
1.4-Dihydro-2.6-dimethyl-4-(2'-nitrophenyl)-pyridin-3.5-dicarbonsäure-isopropyl-(2-methylthioäthyl)-ester,
1.4-Dihydro-2.6-dimethyl-4-(2'-nitrophenyl)-pyridin-3.5-dicarbonsäure-cyclopentyl-(2-methylthioäthyl)-ester,
1.4-Dihydro-2.6-dimethyl-4-(2'-nitrophenyl)-pyridin-3.5-dicarbonsäure-(2-methoxyäthyl)-(2-äthylthioäthyl)-ester,
1.4-Dihydro-2.6-dimethyl-4-(3'-nitrophenyl)-pyridin-3.5-dicarbonsäure-methyl-(2-benzylthioäthyl)-ester,
1.4-Dihydro-2.6-dimethyl-4-(3'-nitrophenyl)-pyridin-3.5-dicarbonsäure-isopropyl-(2-phenylsulfonyläthyl)-ester,
1.4-Dihydro-2.6-dimethyl-4-(2'-trifluormethylphenyl)-pyridin-3.5-dicarbonsäure-äthyl-(2-äthylthioäthyl)-ester,
1.4-Dihydro-2.6-dimethyl-4-(2'-trifluormethylphenyl)-pyridin-3.5-dicarbonsäure-isobutyl-(2-methylthioäthyl)-ester,
1.4-Dihydro-2.6-dimethyl-4-(2'-trifluormethylphenyl)-pyridin-3.5-dicarbonsäure-di-(2-methylthioäthyl)-ester,
1.4-Dihydro-2.6-dimethyl-4-(3'-trifluormethylphenyl)-pyridin-3.5-dicarbonsäure-di-(2-methylsulfonyläthyl)-ester,
1.4-Dihydro-2.6-dimethyl-4-(2'-cyanophenyl)-pyridin-3.5-dicarbonsäure-methyl-(2-methylthioäthyl)-ester,
1.4-Dihydro-2.6-dimethyl-4-(2'-chlorphenyl)-pyridin-3.5-dicarbonsäure-äthyl-(2-methylthioäthyl)-ester,
1.4-Dihydro-2.6-dimethyl-4-(3'-methoxyphenyl)-pyridin-3.5-dicarbonsäure-isopropyl-(2-cyclohexylthioäthyl)-ester,
1.4-Dihydro-2.6-dimethyl-4-(3'-nitrophenyl)-3-(4-chlorphenylsulfonyl)-pyridin-5-carbonsäure-(2-methylthioäthyl)-ester,
1.4-Dihydro-2.6-dimethyl-4-(pyridyl-2)-pyridin-3.5-dicarbonsäure-methyl-(2-methylthioäthyl)-ester,
1.4-Dihydro-2.6-dimethyl-4-(thienyl-2)-pyridin-3.5-dicarbonsäure-

BAD ORIGINAL



isopropyl-(2-methylthioäthyl)-ester,
1.4-Dihydro-2.6-dimethyl-4-(furyl-2)-pyridin-3.5-dicarbonsäure-(2-propoxyäthyl)-(2-äthylthioäthyl)-ester,
1.4-Dihydro-2.6-dimethyl-4-(chinazolinyl-4)-pyridin-3.5-dicarbonsäure-äthyl-(2-benzylthioäthyl)-ester,
1.4-Dihydro-2.6-dimethyl-4-(naphthyl-1)-pyridn-3.5-dicarbonsäure-äthyl-(2-methylthioäthyl)-ester.

Die neuen Verbindungen haben ein breites und vielseitiges pharmakologisches Wirkungsspektrum.

Im einzelnen konnten im Tierexperiment folgende Hauptwirkungen nachgewiesen werden:

1) Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen Nitritähnlichen herzentlastenden Effekt verstärkt.

   Sie beeinflussen bzw. verändern den Herzstoffwechsel im Sinne einer Energieersparnis.

2) Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3) Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z.B. dem Zentralnervensystem) manifestieren.

4) Die Verbindungen senken den Blutdruck von normotonen und hypertonen Tieren und können somit als antihypertensive Mittel verwendet werden.

5) Die Verbindungen haben stark muskulär-spasmolytische Wirkungen, dies an der glatten Muskulatur des Magens, Darmtraktes, des Urogenitaltraktes und des Respirationssystems deutlich werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser nichttoxische organische Lösungsmittel, wie Paraffine (z.B. Erdölfraktionen), pflanzliche Öle (z.B. Erdnuß-/Sesamöl), Alkohole (z.B. Äthylalkohol, Glycerin), Glykole (z.B. Propylenglykol, Polyäthylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel (z.B. Polyoxyäthylen-Fettsäure-Ester, Polyoxyäthylen-Fettalkohol-Äther, Alkylsulfonate und Arylsulfonate), Dispergiermittel (z.B. Lignin, Sulfitablaufen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

BAD ORIGINAL

BAD ORIGINAL



Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch auf Grund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Von besonderem Interesse sind Verbindungen der allgemeinen Formel (I), in welcher

BAD ORIGINAL



R für einen Phenylrest steht der gegebenenfalls substituiert ist durch Halogen, Nitro, Cyano, Trifluormethyl, Trifluormethoxy, Alkyl oder Alkoxy mit je 1 bis 2 Kohlenstoffatomen oder für einen Pyridylrest steht,

$R^1$ und $R^3$ gleich oder verschieden sind und für Alkyl mit 1 oder 2 Kohlenstoffatomen, einen Phenyl oder einen Benzylrest stehen,

$R^2$ für Wasserstoff, Alkyl mit 1 bis 4 Kohlenstoffatomen, insbesondere Methyl, oder Benzyl steht,

Y für eine Alkylengruppe mit 1 bis 4 Kohlenstoffatomen insbesondere für eine Äthylengruppe steht,

n 0 oder 2 bedeutet,

Z für Alkyl mit 1 bis 4 Kohlenstoffatomen steht welches gegebenenfalls durch ein Sauerstoffatom in der Kette unterbrochen ist oder für eine Benzylgruppe steht,

X für die Gruppe $-COR^4$ oder $-COOR^5$ steht, wobei $R^4$ Alkyl mit 1 bis 4 Kohlenstoffatomen, insbesondere Methyl, bedeutet und wobei $R^5$ für geradkettiges, verzweigtes oder cyclisches Alkyl, welches gegebenenfalls durch Sauerstoff, Schwefel oder eine N-Alkyl-Gruppe (1 bis 2 C-Atome) unterbrochen ist, steht, wobei diese Alkylkette bis zu 6 Kohlenstoffatome enthält,

oder für Alkenyl mit bis zu 4, insbesondere mit 3 Kohlenstoffatomen steht,
oder für einen Alkylrest mit 1 bis 4, insbesondere 1 oder 2 Kohlenstoffatomen steht, welche substituiert ist durch Phenyl oder Pyridyl,

oder X für die Gruppe $-S(O)_n-R^6$ steht, wobei n 0 oder 2 bedeutet und $R^6$ für Alkyl mit 1 bis 4 Kohlenstoffen oder für Phenyl steht.

BAD ORIGINAL

BAD ORIGINAL



## Herstellungsbeispiele

### Beispiel 1

3-Acetyl-1.4-dihydro-2.6-dimethyl-4-(3'-nitrophenyl)-pyridin-5-carbonsäure-(2-äthylthioäthyl)-ester

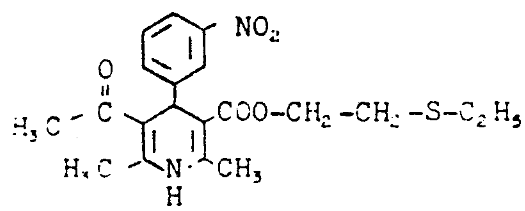

Verfahrensvariante A

17,5 g (75 m Mol) 3'-Nitrobenzylidenacetylaceton wurden zusammen mit 14,2 g (75 m Mol) β-Aminocrotonsäure-(2-äthylthioäthyl)-ester in 80 ml Aethanol 24 Stdn. unter Rückfluß erhitzt. Nach dem Erkalten der Reaktionsmischung wurde das Lösungsmittel im Vakuum abdestilliert und der feste Rückstand aus Aethanol umkristallisiert.
Schmelzpunkt: 130°C; Ausbeute : 21,3 g (70%).

### Beispiel 2

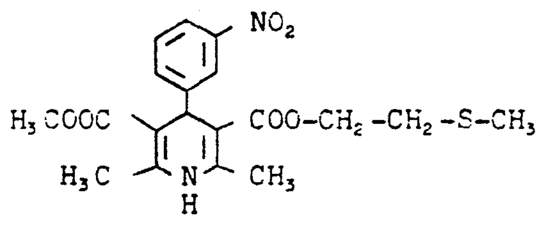

Analog Beispiel 1 wurde durch Umsetzung von 3'-Nitrobenzylidenacetessigsäuremethylester und β-Aminocrotonsäure-(2-methylthioäthyl)-ester in Aethanol der 1.4-Dihydro-2.6-dimethyl-

-4-(3'-nitrophenyl)-pyridin-3.5-dicarbonsäure-methyl-(2-methylthioäthyl)-ester vom Fp.: 126°C (Aethanol) erhalten.
Ausbeute : 80% d.Th.

Beispiel 3

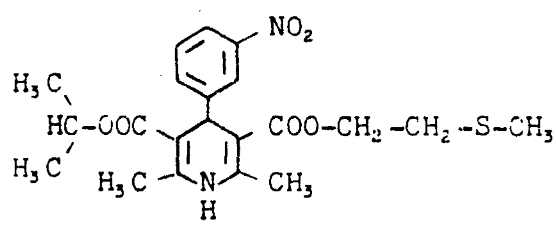

Analog Beispiel 1 wurde durch Umsetzung von 3'-Nitrobenzylidenacetessigsäureisopropylester und β-Aminocrotonsäure-(2-methylthioäthyl)-ester in Aethanol der 1.4-Dihydro-2.6-dimethyl-4-(3'-nitrophenyl)-pyridin-3.5-dicarbonsäure-isopropyl-(2-methylthioäthyl)-ester vom Fp. 154°C (Aethanol) erhalten.
Ausbeute : 84% d.Th.

Beispiel 4

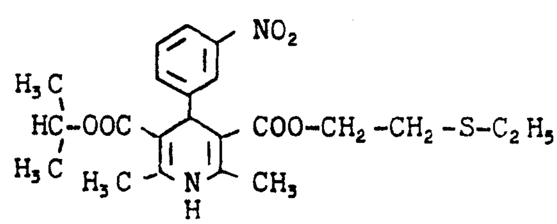

Analog Beispiel 1 wurde durch Umsetzung von 3'-Nitrobenzylidenacetessigsäureisopropylester und β-Aminocrotonsäure-(2-äthylthioäthyl)-ester in Aethanol der 1.4-Dihydro-2.6-dimethyl-4-(3'-nitrophenyl(-pyridin-3.5-dicarbonsäure-isopropyl-(2-äthylthioäthyl)-ester vom Fp.: 95°C (Aethanol) erhalten.
Ausbeute : 70% d.Th.

BAD ORIGINAL



Beispiel 5

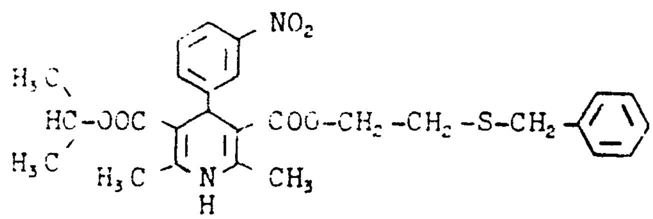

Eine Lösung von 20,8 g(75mMol)3'-Nitrobenzylidenacetessigsäureisopropylester und 18,8 g(75mMol)β-Aminocrotonsäure-(2-benzyl-thioäthyl)-ester in 100 ml Aethanol wurde 24 Stdn. zum Sieden erhitzt. Nach dem Erkalten der Reaktionsmischung wurde das Lösungsmittel i.Vak. abdestilliert und der ölige Rückstand säulenchromatographisch über Kieselgel mit Chloroform als Laufmittel gereinigt. Es wurde ein Oel erhalten (16,3 g = 43%), das auf Grund der spektroskopischen Daten eindeutig als 1.4-Dihydro-2.6-dimethyl-4-(3'-nitrophenyl)-pyridin-3.5-dicarbonsäure-isopropyl-(2-benzylthioäthyl)-ester identifiziert wurde.

Beispiel 6

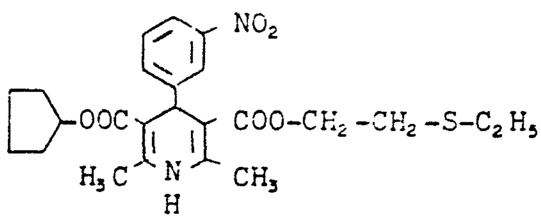

Analog Beispiel 1 wurde durch Umsetzung von 3'-Nitrobenzylidenacetessigsäurecyclopentylester und β-Aminocrotonsäure-(2-äthylthioäthyl)-ester in Aethanol der 1.4-Dihydro-2.6-dimethyl-4-(3'-nitrophenyl)-pyridin-3.5-dicarbonsäure-cyclopentyl-(2-äthylthioäthyl)-ester vom Fp.: 93°C (Aethanol) erhalten. Ausbeute : 68% d.Th.

Beispiel 7

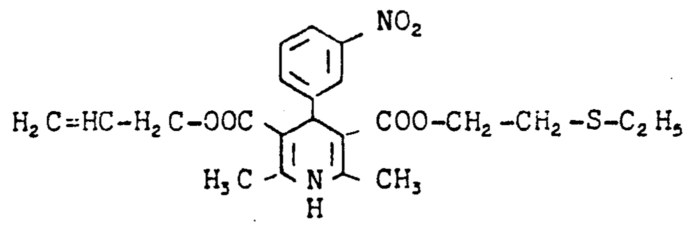

Analog Beispiel 5 wurde durch Umsetzung von 3'-Nitrobenzyliden-acetessigsäureallylester und β-Aminocrotonsäure-(2-äthylthio-äthyl)-ester in Aethanol der 1.4-Dihydro-2.6-dimethyl-4-(3'-nitrophenyl)-pyridin-3.5-dicarbonsäure-allyl-(2-äthylthio-äthyl)-ester (Oel) erhalten.
Ausbeute : 80% d.Th.

Beispiel 8

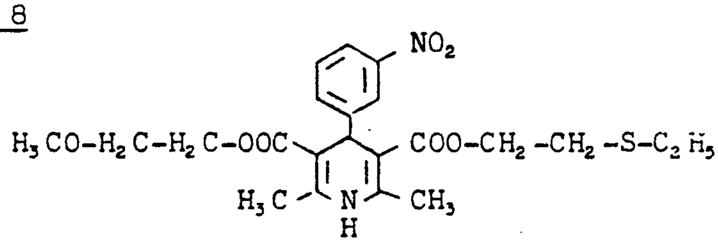

Analog Beispiel 1 wurde durch Umsetzung von 3'-Nitrobenzyliden-acetessigsäure-(2-methoxyäthyl)-ester und β-Aminocrotonsäure-(2-äthylthioäthyl)-ester in Aethanol der 1.4-Dihydro-2.6-dimethyl-4-(3'-nitrophenyl)-pyridin-3.5-dicarbonsäure-(2-methoxyäthyl)-(2-äthylthioäthyl)-ester vom Fp.: 90 °C (Aethanol) erhalten.
Ausbeute : 79% d.Th.

BAD ORIGINAL



Beispiel 9

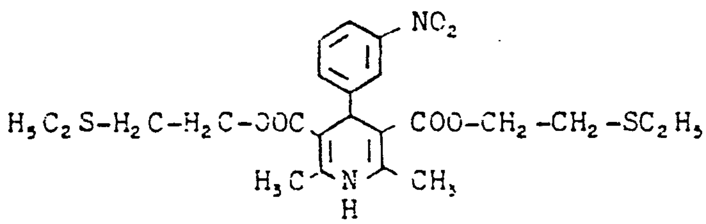

Analog Beispiel 1 wurde durch Umsetzung von 3'-Nitrobenzylidenacetessigsäure-(2-äthylthioäthyl)-ester und β-Aminocrotonsäure-(2-äthylthioäthyl)-ester in Aethanol der 1.4-Dihydro-2.6-dimethyl-4-(3'-nitrophenyl)-pyridin-3.5-dicarbonsäure-di-(2-äthylthioäthyl)-ester vom Fp. : 118°C (Aethanol) erhalten.
Ausbeute : 60% d.Th.

Beispiel 10

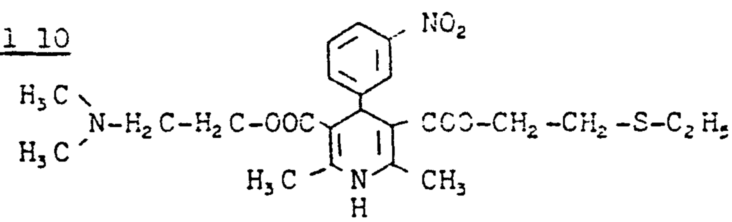

Analog Beispiel 5 wurde durch Umsetzung von 3'-Nitrobenzylidenacetessigsäure-(2-dimethylaminoäthyl)-ester und β-Aminocrotonsäure-(2-äthylthioäthyl)-ester in Aethanol der 1.4-Dihydro-2.6-dimethyl-4-(3'-nitrophenyl)-pyridin-3.5-dicarbonsäure-(2-dimethylaminoäthyl)-(2-äthylthioäthyl)-ester erhalten (Oel, Eluierungsmittel Chloroform : Aceton = 20 : 1).
Ausbeute : 48% d. Th.

BAD ORIGINAL



Beispiel 11

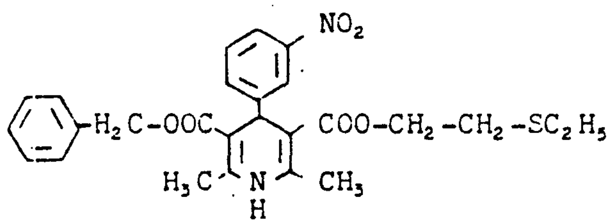

Analog Beispiel 1 wurde durch Umsetzung von 3'-Nitrobenzylidenacetessigsäurebenzylester und β-Aminocrotonsäure-(2-äthylthioäthyl)-ester in Aethanol der 1.4-Dihydro-2.6-dimethyl-4-(3'-nitrophenyl)-pyridin-3.5-dicarbonsäure-benzyl-(2-äthylthioäthyl)-ester vom Fp. : 132°C (Aethanol) erhalten.
Ausbeute : 69% d.Th.

Beispiel 12

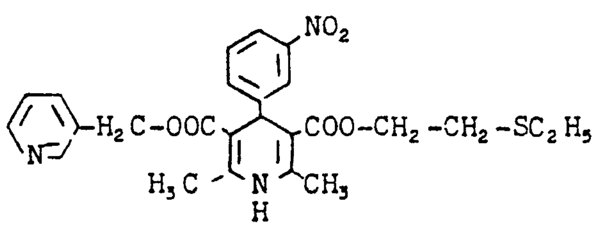

Analog Beispiel 1 wurde durch Umsetzung von 3'-Nitrobenzylidenacetessigsäure-(pyridyl-3-methyl)-ester und β-Aminocrotonsäure-(2-äthylthioäthyl)-ester in Aethanol der 1.4-Dihydro-2.6-dimethyl-4-(3'-nitrophenyl)-pyridin-3.5-dicarbonsäure-(pyridyl-3-methyl)-(2-äthylthioäthyl)-ester vom Fp. : 144°C (Aethanol) erhalten.
Ausbeute : 65% d. Th.

BAD ORIGINAL



Beispiel 13

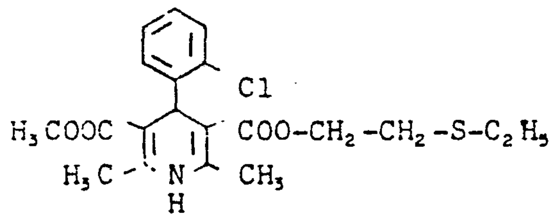

Analog Beispiel 1 wurde durch Umsetzung von 2'-Chlorbenzylidenacetessigsäuremethylester und β-Aminocrotonsäure-(2-äthylthioäthyl)-ester in Aethanol der 1.4-Dihydro-2.6-dimethyl-4-(2'-chlorphenyl)-pyridin-3.5-dicarbonsäure-methyl-(2-äthylthioäthyl)-ester vom Fp. : 92°C (Petroläther/Aether) erhalten.
Ausbeute : 71% d.Th.

Beispiel 14

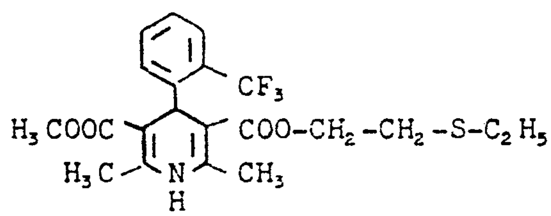

Analog Beispiel 5 wurde durch Umsetzung von 2'-Trifluormethylbenzylidenacetessigsäuremethylester und β-Aminocrotonsäure-(2-äthylthioäthyl)-ester in Aethanol der 1.4-Dihydro-2.6-dimethyl-4-(2'-trifluormethylphenyl)-pyridin-3.5-dicarbonsäuremethyl-(2-äthylthioäthyl)-ester (Oel) erhalten.
Ausbeute : 65% d.Th.

BAD ORIGINAL



Beispiel 15

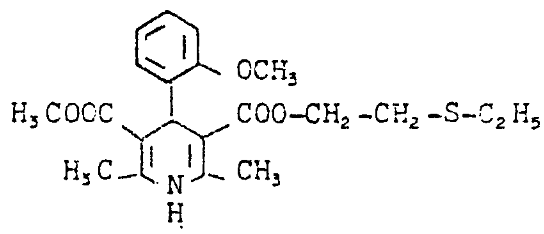

Analog Beispiel 1 wurde durch Umsetzung von 2'-Methoxybenzyliden acetessigsäuremethylester und β-Aminocrotonsäure-(2-äthylthioäthyl)-ester in Aethanol der 1.4-Dihydro-2.6-dimethyl-4-(2'-methoxyphenyl)-pyridin-3.5-dicarbonsäuremethyl-(2-äthylthioäthyl)-ester vom Fp. : 121°C (Aethanol) erhalten.
Ausbeute : 83% d.Th.

Beispiel 16

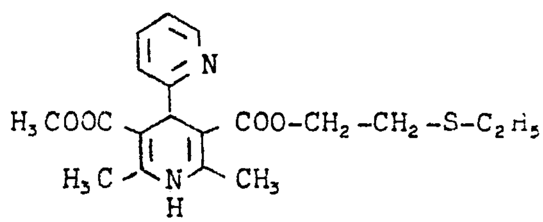

Analog Beispiel 1 wurde durch Umsetzung von 2-Acetyl-3-(pyridyl-2)-acrylsäuremethylester und β-Aminocrotonsäure-(2-äthylthioäthyl)-ester in Aethanol der 1.4-Dihydro-2.6-dimethyl-4-(pyridyl-2)-pyridin-3.5-dicarbonsäure-methyl-(2-äthylthioäthyl)-ester vom Fp. : 137°C (Aethanol) erhalten.
Ausbeute : 63% d.Th.

BAD ORIGINAL



Beispiel 17

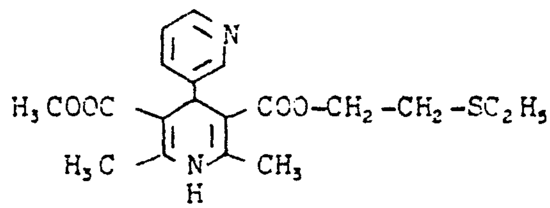

Analog Beispiel 1 wurde durch Umsetzung von 2-Acetyl-3-(pyridyl-3)-acrylsäuremethylester und β-Aminocrotonsäure-(2-äthylthioäthyl)-ester in Aethanol der 1.4-Dihydro-2.6-dimethyl-4-(pyridyl-3)-pyridin-3.5-dicarbonsäure-methyl-(2-äthylthioäthyl)-ester vom Fp.: 151°C (Aethanol) erhalten.
Ausbeute : 72% d. Th.

Beispiel 18

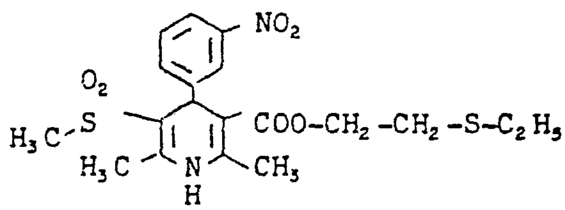

Analog Beispiel 1 wurde durch Umsetzung von 3-Methylsulfonyl-4-(3'-nitrophenyl)-3-butenon-2-on und β-Aminocrotonsäure-(2-äthylthioäthyl)-ester in Aethanol der 1.4-Dihydro-2.6-dimethyl-4-(3'-nitrophenyl)-3-methylsulfonyl-pyridin-5-carbonsäure-(2-äthylthioäthyl)-ester vom Fp. : 136°C (Aethanol) erhalten.
Ausbeute : 63% d.Th.

BAD ORIGINAL



Beispiel 19

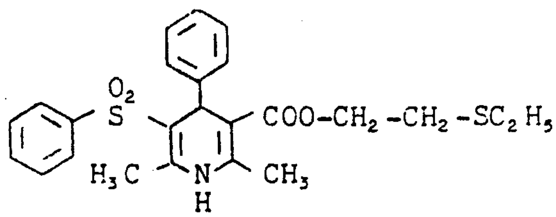

Analog Beispiel 1 wurde durch Umsetzung von 3-Phenylsulfonyl-4-phenyl-3-buten-2-on und β-Aminocrotonsäure-(2-äthylthioäthyl)-ester ind Aethanol der 1.4-Dihydro-2.6-dimethyl-4-phenyl-3-phenylsulfonyl-pyridin-5-carbonsäure-(2-äthylthioäthyl)-ester vom Fp. : 129°C (Aethanol) erhalten.
Ausbeute : 72% d.Th.

Beispiel 20

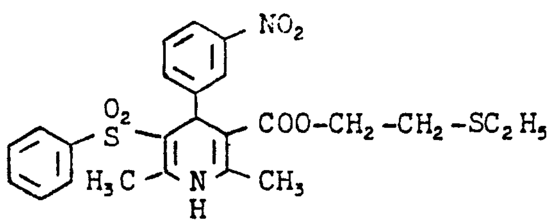

Analog Beispiel 1 wurde durch Umsetzung von 3-Phenylsulfonyl-4-(3'-nitrophenyl)-3-buten-2-on und β-Aminocrotonsäure-(2-äthylthioäthyl)-ester in Aethanol der 1.4-Dihydro-2.6-dimethyl-4-(3'-nitrophenyl)-3-phenylsulfonyl-pyridin-5-carbonsäure-(2-äthylthioäthyl)-ester vom Fp. : 130°C (Aethanol) erhalten.
Ausbeute : 70% d.Th.

BAD ORIGINAL



Beispiel 21

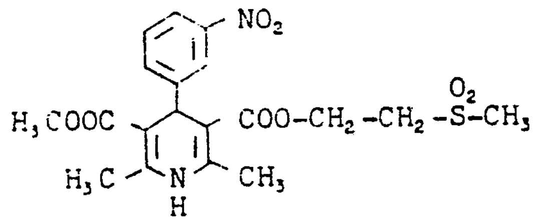

Analog Beispiel 1 wurde durch Umsetzung von 3'-Nitrobenzylidenacetessigsäuremethylester und β-Aminocrotonsäure-(2-methylsulfonyläthyl)-ester in Aethanol der 1.4-Dihydro-2.6-dimethyl-4-(3'-nitrophenyl)-pyridin-3.5-dicarbonsäure-methyl-(2-methylsulfonyläthyl)-ester vom Fp.: 74°C (Aethanol) erhalten.
Ausbeute : 68% d. Th.

Beispiel 22

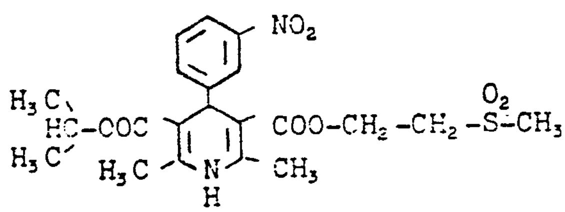

Analog Beispiel 1 wurde durch Umsetzung von 3'-Nitrobenzylidenacetessigsäureisopropylester und β-Aminocrotonsäure-(2-methylsulfonyläthyl)-ester in Aethanol der 1.4-Dihydro-2.6-dimethyl-4-(3'-nitrophenyl)-pyridin-3.5-dicarbonsäure-isopropyl-(2-methylsulfonyläthyl)-ester vom Fp.: 153°C (Aethanol) erhalten.
Ausbeute : 62% d.Th.

BAD ORIGINAL



Patentansprüche

1. 1,4-Dihydropyridin mit schwefelhaltigen Estergruppierungen der Formel (I)

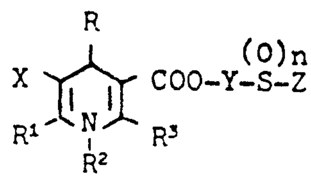

(I)

in welcher

R für einen Arylrest oder für einen Thienyl-, Furyl-, Pyrryl-, Pyrazolyl-, Imidazolyl-, Oxazolyl-, Isoxazolyl-, Thiazolyl-, Pyridyl-, Pyridazinyl-, Pyrimidyl-, Pyrazinyl-, Chinolyl-, Isochinolyl-, Indolyl- Benzimidazolyl-, Chinazolyl- oder Chinoxalylrest steht, wobei der Arylrest sowie die Heterocyclen gegebenenfalls 1 bis 3 gleiche oder verschiedene Substituenten aus der Gruppe Phenyl, Alkyl, Alkenyl, Alkinyl, Alkoxy, Alkenoxy, Alkinoxy, Alkylen, Dioxyalkylen, Halogen, Trifluormethyl, Trifluormethoxy, Hydroxy, Amino, Alkylamino, Nitro, Cyano, Azido, Carboxy, Carbalkoxy, Carbonamido, Sulfonamido oder $SO_m$-Alkyl ($m$ = 0 bis 2) enthalten,

$R^1$ und $R^3$ gleich oder verschieden sind und für Wasserstoff, einen geradkettigen oder verzweigten Alkylrest, einen Arylrest oder einen Aralkylrest stehen,

$R^2$ Wasserstoff oder einen geradkettigen oder verzweigten

Alkylrest bedeutet, der gegebenenfalls durch ein oder zwei Sauerstoffatome in der Alkylkette unterbrochen ist, oder für einen Aryl- oder Aralkylrest steht,

Y eine gegebenenfalls durch Alkyl substituierte Alkylengruppe darstellt,

n 0, 1 oder 2 bedeutet,

Z für einen geradkettigen, verzwiegten oder cyclischen, gesättigten oder ungesättigten aliphatischen Kohlenwasserstoffrest steht, der getebenenfalls durch ein oder zwei Sauerstoffatome in der Kette unterbrochen ist und/oder der gegebenenfalls substituiert ist durch eine Phenoxy- oder Phenylgruppe, welche ihrerseits gegebenenfalls substituiert sind durch Halogen, Cyano, Dialkylamino, Alkyl, Alkoxy, Trifluormethyl oder Nitro, oder der gegebenenfalls substituiert ist durch eine α-, ß- oder γ-Pyridylgruppe, und

X für die Gruppe $-COR^4$, worin $R^4$ gegebenenfalls substituiertes Alkyl, Aryl oder Aralkyl oder eine Amino-, Monoalkylamino- oder Dialkylaminogruppe bedeutet,
oder
für die Gruppe $-COOR^5$, wobei $R^5$ einen geradkettigen, verzweigten oder cyclischen, gesättigten oder ungesättigten Kohlenwasserstoffrest darstellt, der gegebenenfalls durch 1 oder 2 Sauerstoffatome in der Kette unterbrochen ist, und/oder der gegebenenfalls substituiert ist durch eine Phenyl-, Phenoxy-, Phenylthio- oder Phenylsulfonylgruppe, welche ihrerseits wieder durch Halogen, Cyano, Dialkylamino, Alkoxy, Alkyl, Trifluormethyl oder Nitro substituiert sein können,

oder der gegebenenfalls durch eine α-, ß- oder γ-Pyridylgruppe oder durch eine Aminogruppe substituiert ist, wobei diese Aminogruppe zwei gleiche oder verschiedene Substituenten aus der Gruppe Alkyl, Alkoxyalkyl, Aryl und Aralkyl trägt und wobei diese Substituenten gegebenenfalls mit dem Stickstoffatom einen 5- bis 7-gliedrigen Ring bilden, der als weiteres Heteroatom ein Sauerstoff- oder Schwefelatom oder die N-Alkyl-Gruppierung enthalten kann,

oder

für die Gruppe $-COO-Y'-S(O)_{n}'-Z'$, wobei diese Gruppe mit $-COO-Y-S(O)_{n}-Z$ gleich oder verschieden sein kann und wobei die Definitionen von Y', n' und Z' denen von Y, n und Z entsprechen,

oder

für die Gruppe $-S(O)_{r}-R^{6}$ steht, in der r = 0, 1 oder 2 bedeutet und $R^{6}$ einen geradkettigen, verzweigten oder cyclischen, gesättigten oder ungesättigten aliphatischen Kohlenwasserstoffrest darstellt, der gegebenenfalls durch 1 oder 2 Sauerstoffatome in der Kette unterbrochen ist und/oder der durch eine gegebenenfall durch Halogen, Cyano, Dialkylamino, Alkoxy, Alkyl, Trifluormethyl oder Nitro substituierte Phenyl-, Phenoxy-, Phenylthio- oder Phenylsulfonylgruppe oder durch eine α-, β- oder γ-Pyridiylgruppe oder durch eine Aminogruppe substituiert sein kann, wobei diese Aminogruppe zwei gleiche oder verschiedene Substituenten aus der Gruppe Alkyl, Alkoxyalkyl, Aryl und Aralkyl trägt und wobei diese Substituenten gegebenenfalls mit dem Stickstoffatom einen 5- bis 7-gliedrigen Ring bilden, der als weiteres Heteroatom ein Sauerstoff-

BAD ORIGINAL

oder Schwefelatom oder die N-Alkyl-Gruppierung enthalten kann,
oder in der $R^6$
für einen Arylrest steht, der gegebenenfalls 1 bis 3 gleiche oder verschiedene Substituenten aus der Gruppe Alkyl, Alkoxy, Halogen, Cyano, Trifluormethyl, Trifluormethoxy, Dialkylamino oder Nitro enthält.

2. Verfahren zur Herstellung von 1,4-Dihydropyridinen mit schwefelhaltigen Estergruppierungen gemäß Anspruch 1, dadurch gekennzeichnet, daß man

A) Ylidenverbindungen der Formel II,

$$R-CH=C\begin{matrix}\nearrow CO-R^1\\ \searrow X\end{matrix} \qquad (II)$$

in welcher
R, $R^1$ und X die oben angegebene Bedeutung haben,
mit Enaminocarbonsäureestern der Formel III,

$$R^3-\underset{R^2NH}{\underset{|}{C}}=CH-COO-Y-S(O)_n-Z \qquad (III)$$

in welcher
$R^2$, $R^3$, Y, Z und n die oben angegebene Bedeutung haben, gegebenenfalls in Gegenwart von inerten organischen Lösungsmitteln umsetzt,
oder

B) Ylidenverbindungen der Formel II,

BAD ORIGINAL



$$R-CH=C\begin{matrix}\swarrow CO-R^1 \\ \searrow X\end{matrix} \qquad (II)$$

in welcher R, $R^1$ und X die oben angegebene Bedeutung haben, mit Aminen der Formel IV und β-Ketocarbonsäureestern der Formel V,

$R^2-NH_2$ $\qquad$ $R^3-CO-CH_2-COO-Y-S(O)_n-Z$

(IV) $\qquad$ (V)

in welcher $R^2$, $R^3$, Y, Z und n die oben angegebene Bedeutung haben, gegebenenfalls in Gegenwart inerter organischer Lösungsmittel umsetzt,
oder

C) Yliden-β-dicarbonylverbindungen der Formel VI,

$$R-CH=C\begin{matrix}\swarrow CO-R^3 \\ \searrow COO-Y-S(O)_n-Z\end{matrix} \qquad (VI)$$

in welcher R, $R^3$, Y, Z und n die oben angegebene Bedeutung haben, mit Enaminoverbindungen der Formel VII,

$$\begin{matrix} R^1-C=CH-X \\ | \\ R^2-NH \end{matrix} \qquad (VII)$$

in welcher $R^1$, $R^2$ und X die oben angegebene Bedeutung haben,

gegebenenfalls in Gegenwart inerter organischer Lösungsmittel zu Reaktion bringt,
oder

D) Yliden-β-dicarbonylverbindungen der Formel VI,

$$R-CH=C\begin{matrix}CO-R^3\\COO-Y-S(O)_n-Z\end{matrix} \qquad (VI)$$

in welcher
R, $R^3$, Y, Z und n die oben angegebene Bedeutung haben, mit Aminen der Formel IV und Keto-Derivaten der Formel VIII,

$$R^2-NH_2 \quad (IV) \qquad\qquad R^1-CO-CH_2-X \quad (VIII)$$

in welchen
$R^2$, $R^1$ und X die oben angegebene Bedeutung haben, gegebenenfalls in Gegenwart inerter organischer Lösungsmittel umsetzt,
oder

E) Aldehyde der Formel IX,

$$R-C\begin{matrix}H\\O\end{matrix} \qquad (IX)$$

in welcher
R die oben angegebene Bedeutung hat, mit Enaminoverbindungen der Formel VII,

$$\begin{matrix}R^1-C=CH-X\\R^2-NH\end{matrix} \qquad (VII)$$

BAD ORIGINAL



in welcher
$R^1$, $R^2$ und X die oben angegebene Bedeutung haben, und β-Ketocarbonsäureestern der Formel V,

$$R^3-CO-CH_2-COO-Y-S(O)_n-Z \qquad (V)$$

in welcher
$R^3$, Y, Z und n die oben angegebene Bedeutung haben, gegebenenfalls in Gegenwart inerter organischer Lösungsmittel umsetzt,
oder

F) Aldehyde der Formel IX,

$$R-C{\overset{H}{\underset{O}{\lessgtr}}} \qquad (IX)$$

in welcher
R die oben angegebene Bedeutung hat,
mit Enaminocarbonsäureester der Formel III,

$$\underset{R^2NH}{R^3-C}=CH-COO-Y-S(O)_n-Z \qquad (III)$$

in welcher
$R^2$, $R^3$, Y, Z und n die oben angegebene Bedeutung haben, und Keto-Derivaten der Formel VIII,

$$R^1-CO-CH_2-X \qquad (VIII)$$

in welcher
$R^1$ und X die oben angegebene Bedeutung haben, gegebenenfalls in Gegenwart inerter organischer Lösungsmitte

bei Temperaturen zwischen 20°C und 150°C umsetzt.

BAD ORIGINAL



3. 1,4-Dihydropyridine mit schwefelhaltigen Estergruppierungen der allgemeinen Formel (I) in welcher

R für einen Phenylrest steht der gegebenenfalls substituiert ist durch Halogen, Nitro, Cyano, Trifluormethyl, Trifluormethoxy, Alkyl oder Alkoxy mit je 1 bis 2 Kohlenstoffatomen oder für einen Pyridylrest steht,

$R^1$ und $R^3$ gleich oder verschieden sind und für Alkyl mit 1 oder 2 Kohlenstoffatomen, einen Phenyl oder einen Benzylrest stehen,

$R^2$ für Wasserstoff, Alkyl mit 1 bis 4 Kohlenstoffatomen, insbesondere Methyl, oder Benzyl steht,

Y für eine Alkylengruppe mit 1 bis 4 Kohlenstoffatomen insbesondere für eine Äthylengruppe steht,

n 0 oder 2 bedeutet,

Z für Alkyl mit 1 bis 4 Kohlenstoffatomen steht welches gegebenenfalls durch ein Sauerstoffatom in der Kette unterbrochen ist oder für eine Benzylgruppe steht,

X für die Gruppe $-COR^4$ oder $-COOR^5$ steht, wobei $R^4$ Alkyl mit 1 bis 4 Kohlenstoffatomen, insbesondere Methyl, bedeutet und wobei $R^5$ für geradkettiges, verzweigtes oder cyclisches Alkyl, welches gegebenenfalls durch Sauerstoff, Schwefel oder eine N-Alkyl-Gruppe (1 bis 2 C-Atome) unterbrochen ist, steht, wobei diese Alkylkette bis zu 6 Kohlenstoffatome enthält,

oder für Alkenyl mit bis zu 4, insbesondere mit 3 Kohlenstoffatomen steht,
oder für einen Alkylrest mit 1 bis 4, insbesondere 1 oder 2 Kohlenstoffatomen steht, welche substituiert ist durch Phenyl oder Pyridyl,

oder X für die Gruppe $-S(O)_n-R^6$ steht, wobei n O oder 2 bedeutet und $R^6$ für Alkyl mit 1 bis 4 Kohlenstoffen oder für Phenyl steht.

4. Arzneimittel enthaltend mindestens ein 1,4-Dihydropyridin mit schwefelhaltiger Estergruppierung gemäß Anspruch 1.

5. Verwendung von 1,4-Dihydropyridinen mit schwefelhaltigen Estergruppierungen gemäß Anspruch 1 bei der Bekämpfung von Erkrankungen.

6. Verwendung von 1,4-Dihydropyridinen mit schwefelhaltigen Estergruppierungen bei der Bekämpfung von Kreislauferkrankungen.

7. Verfahren zur Herstellung von Arzneimitteln, dadurch gekennzeichnet, daß man 1,4-Dihydropyridine mit schwefelhaltigen Estergruppierungen gemäß Anspruch 1 gegebenenfalls unter Zusatz von inerten pharmazeutisch unbedenklichen Hilfs- und Trägerstoffen in eine geeignete Applikationsform überführt.

8. Verfahren zur Behandlung von Kreislauferkrankungen, dadurch gekennzeichnet, daß man 1,4-Dihydropyridine mit schwefelhaltigen Estergruppierungen gemäß Anspruch 1 Menschen oder Tieren im Bedarfsfalle appliziert.

0001769

BAD ORIGINAL

Europäisches Patentamt

# EUROPÄISCHER TEILRECHERCHENBERICHT,

der nach Regel 45 des Europäischen Patent-übereinkommens für das weitere Verfahren als europäischer Recherchenbericht gilt

Nummer der Anmeldung

EP 78 101 135.8

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| - | DE - A - 2 629 892 (FUJISAWA)<br>*Anspruch 98*<br>-- | 2 | C 07D 211/90<br>A 61K 31/44<br>C 07D 401/04<br>C 07D 401/12//<br>C 07D 401/00<br>C 07D 405/00<br>C 07D 409/00<br>C 07D 413/00<br>C 07D 417/00 |
| - | DE - A - 2 508 181 (BAYER)<br>*Ansprüche 2 bis 5*<br>-- | 2,4-7 | |
| - | DE - A - 2 524 277(CIBA-GEIGY)<br>*Ansprüche 1, 41*<br>-- | 2,7 | RECHERCHIERTE SACHGEBIETE (Int. Cl.²) |
| P | DE - A - 2 616 995 (BAYER)<br>*Anspruch 1*<br>-- | 2 | A 61K 31/44<br>C 07D 211/90<br>C 07D 401/04<br>C 07D 401/12 |
| DA | Naturwissenschaften, 58. Jahrgang, 1971<br>Berlin, Heidelberg, New York<br>F. BOSSERT u.a. "Dihydropyridine, eine neue Gruppe stark wirksamer Coronartherapeutika"<br>Seite 578 | | |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7

Unvollständig recherchierte Patentansprüche:

Nicht recherchierte Patentansprüche: 8

Grund für die Beschränkung der Recherche

Verfahren zur therapeutischen Behandlung des menschlichen oder tierischen Körpers.

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Berlin | 26-01-1979 | FROELICH |

EPA Form 1505.1 06.78